# EUROPÄISCHE PATENTSCHRIFT

(11) **EP 3 829 434 B1**
(45) Veröffentlichungstag und Bekanntmachung des Hinweises auf die Patenterteilung: **25.10.2023**
(21) Anmeldenummer: 19770190.7
(22) Anmeldetag: 25.07.2019
(51) Int. Cl.: A61B 5/087, G01F 1/36

(54) **ATEMSTROMSENSOR**
RESPIRATORY SENSOR
CAPTEUR DE DÉBIT RESPIRATOIRE

(30) Priorität: 27.07.2018 EP 18185994
(43) Veröffentlichungstag der Anmeldung: 09.06.2021
(73) Patentinhaber: imtmedical ag, 9470 Buchs SG (CH)
(72) Erfinder: DAESCHER, Jakob, 7306 Fläsch (CH); FRIBERG, Harri, 9493 Mauren (LI)
(74) Vertreter: PPR AG
(86) Internationale Anmeldenummer: PCT/IB2019/056367
(87) Internationale Veröffentlichungsnummer: WO 2020/021492

(56) Entgegenhaltungen:
- DE-A1-102010 040 287
- DE-U1-202017 102 703
- US-A- 4 993 269
- US-A1- 2009 056 472

## Beschreibung

Die Erfindung betrifft einen Atemstromsensor nach dem Oberbegriff des Anspruchs 1, einen Atemadapter nach dem Oberbegriff des Anspruchs 10 und ein Verfahren zur Herstellung eines Atemstromsensors nach Anspruch 12.

Atemstromsensoren, auch Differenzdruck-Durchflusssensoren, Durchflussmessfühler oder Fluss-Sensoren genannt, werden zwischen einem von einem Beatmungsgerät oder Anästhesiegerät ausgehenden Schlauch und einem dem Patienten zugeführten Schlauch angeordnet.

Aus der US 4,403,514 A ist ein Atemstromsensor bekannt, der ein Strömungsrohr umfasst, das eine Längsachse, einen ersten Strömungsrohrabschnitt und einen zweiten Strömungsrohrabschnitt sowie im Bereich der freien Enden Schlauchanschlüsse für Schläuche aufweist. In dem Strömungsrohr ist zwischen dem ersten Strömungsrohrabschnitt und dem zweiten Strömungsrohrabschnitt ein Strömungswiderstand angeordnet. Weiter sind radial abragende Anschlüsse zur Abnahme der durch den Strömungswiderstand erzeugten Druckdifferenz vorgesehen. An diese Anschlüsse werden Verbindungsschläuche vorgesehen, welche an einer Messvorrichtung anschliessbar sind. Ein Anschluss ist an dem ersten Strömungsrohrabschnitt angeordnet und mündet über eine erste Verbindungsleitung in den ersten Strömungsrohrabschnitt. Ein weiterer Anschluss ist an dem zweiten Strömungsrohrabschnitt angeordnet und mündet über eine zweite Verbindungsleitung in den zweiten Strömungsrohrabschnitt.

Nachteilig an dieser bekannten Lösung ist, dass an den Anschlüssen angeordnete Verbindungsleitungen abknicken können, was unter Umständen zu einer Verfälschung der Messergebnisse führt.

Aus der CH 701 755 B1 ist ein Atemstromsensor bekannt, der ein Strömungsrohr umfasst, das eine Längsachse, einen ersten Strömungsrohrabschnitt und einen zweiten Strömungsrohrabschnitt aufweist. In dem Strömungsrohr ist zwischen dem ersten Strömungsrohrabschnitt und dem zweiten Strömungsrohrabschnitt ein Strömungswiderstand angeordnet. Weiter sind Anschlüsse zur Abnahme der durch den Strömungswiderstand erzeugten Druckdifferenz vorgesehen. Ein erster Anschluss mündet über eine erste Verbindungsleitung in den ersten Strömungsrohrabschnitt und ein weiterer Anschluss mündet über eine zweite Verbindungsleitung in den zweiten Strömungsrohrabschnitt. Die Anschlüsse sind beide an dem ersten Strömungsrohrabschnitt angeordnet. Die Anschlüsse weisen jeweils einen Verbindungsleitungsabschnitt der Verbindungsleitung auf, der parallel zur Längsachse des Strömungsrohrs verläuft. Die Öffnungen der Anschlüsse sind in die gleiche Richtung ausgerichtet.

Nachteilig an dieser bekannten Lösung ist, dass zur Ausbildung der Verbindungsleitungen von den Anschlüssen in die entsprechenden Strömungsrohrabschnitte nicht nur radial, d. h. nach aussen abragende Flanschfortsätze vorgesehen werden müssen, sondern zur Sicherstellung von dichten Verbindungen, aufwändige konstruktive und fertigungstechnische Massnahmen in der Ausgestaltung der Dichtungsmittel erforderlich sind. Nur dadurch lässt sich ein Luftkurzschluss verhindern, der in der Anwendung des Atemstromsensors zu stark verfälschten Messergebnissen führen würde.

Die DE 20 2017 102 703 U1 zeigt einen Durchflusssensor mit einem Strömungsrohr, das eine Längsachse, ein erstes Schlauchelement und ein zweites Schlauchelement aufweist. Weiters weist der Durchflusssensor einen Strömungswiderstand auf, der in dem Strömungsrohr zwischen dem ersten Schlauchelement und dem zweiten Schlauchelement angeordnet ist. An den Schlauchelementen sind Verlängerungsschläuche zur Abnahme der durch den Strömungswiderstand erzeugten Druckdifferenz angeordnet, wobei ein erster Verlängerungsschlauch in das erste Schlauchelement mündet und ein weiterer Verlängerungsschlauch in das zweite Schlauchelement mündet. Der erste Verlängerungsschlauch ist an dem ersten Schlauchelement und der weitere Verlängerungsschlauch ist an dem zweiten Schlauchelement angeordnet.

Nachteilig an dieser Lösung ist, dass die Verlängerungsschläuche vom Durchflusssensor im Wesentlichen radial abragen, sodass im Gebrauch die dort angeordneten Messschläuche ebenfalls im Wesentlichen radial abragen, wodurch dieser Durchflusssensor im Gebrauch verhältnismässig viel Platz einnimmt und somit eine komplizierte Handhabung des Durchflusssensors im Gebrauch für den Benutzer verursacht.

Aus der US 6,585,662 B1 ist ein Atemstromsensor bekannt, der vom konstruktiven Aufbau her nahezu identisch mit einem Atemstromsensor gemäss der CH 701 755 B1 ist, jedoch eine wesentlich in der Anwendung praktischere Ausgestaltung als dieser aufweist.

Aufgabe der vorliegenden Erfindung ist es somit, einen Atemstromsensor zu schaffen, welcher wenigstens einen Teil der vorgenannten Nachteile nicht aufweist, eine sehr kompakte Ausgestaltung aufweist sowie dabei einfach und mit hoher Qualität herstellbar ist.

Die Aufgabe wird durch die Merkmale der unabhängigen Ansprüche gelöst. Vorteilhafte Weiterbildungen sind in den Figuren und in den abhängigen Patentansprüchen dargelegt.

Gemäss der Erfindung umfasst ein Atemstromsensor ein Strömungsrohr, das eine Längsachse, einen ersten Strömungsrohrabschnitt und einen zweiten Strömungsrohrabschnitt aufweist, ein Strömungswiderstand, der in dem Strömungsrohr zwischen dem ersten Strömungsrohrabschnitt und dem zweiten Strömungsrohrabschnitt angeordnet ist, und Anschlüsse zur Abnahme der durch den Strömungswiderstand erzeugten Druckdifferenz, wobei ein erster Anschluss über eine erste Verbindungsleitung in den ersten Strömungsrohrabschnitt mündet und ein weiterer Anschluss über eine zweite Verbindungsleitung in den zweiten Strömungsrohrabschnitt mündet, wobei die Anschlüsse jeweils einen Verbindungsleitungsabschnitt der Verbindungsleitung aufweisen, der im Wesentlichen parallel zur Längsachse des Strömungsrohrs verläuft, und die Öffnungen der Anschlüsse in die gleiche Richtung ausgerichtet sind. Der erste Anschluss ist an dem ersten Strömungsrohrabschnitt und der weitere Anschluss ist an dem zweiten Strömungsrohrabschnitt angeordnet.

Unter dem Begriff "im Wesentlichen parallel zur Längsachse des Strömungsrohrs" wird in diesem Zusammenhang einerseits eine mathematisch definierte parallele Ausrichtung zu dieser Längsachse verstanden, aber auch eine um wenige Grad davon abweichende Ausrichtung.

Vorteilhaft sind die beiden Anschlüsse des Atemstromsensors selbst zueinander im Wesentlichen parallel ausgerichtet, sodass der Atemstromsensor schlank ausgebildet werden kann.

Jeder der Anschlüsse zur Abnahme der durch den Strömungswiderstand erzeugten Druckdifferenz ist an dem jeweiligen Strömungsrohrabschnitt angeordnet, in dessen Inneren die entsprechende Verbindungsleitung mündet. Beim Zusammenführen der Strömungsrohrabschnitte muss daher lediglich der Dichtung des Strömungskanals in dem Strömungsrohr Sorge getragen werden, womit die einzelnen Teile des Atemstromsensors konstruktiv einfacher ausgebildet sowie leicht zusammenführbar sind. Zusätzliche Luftführungen in den Verbindungsleitungen lassen sich vermeiden, womit die Messgenauigkeit gegenüber den bisher bekannten Atemstromsensoren sichergestellt und sogar verbessert ist.

Vorteilhaft sind die Verbindungsleitungen stossstellenfrei ausgebildet. Mit anderen Worten ist bei jeder Verbindungsleitung von den Öffnungen der Anschlüsse bis zur Mündung in den jeweiligen Strömungsabschnitt vorteilhaft keine Stossstelle vorhanden. Damit lassen sich die Messung nachteilig beeinflussende Effekte vermeiden und eine genaue Messgenauigkeit wird zusätzlich verbessert.

Die Anschlüsse zur Abnahme der durch den Strömungswiderstand erzeugten Druckdifferenz können bei Gewährleistung des Anschlusses von Verbindungsschläuche nahe an einer Gehäuseaussenseite des Atemstromsensors angeordnet werden, was eine sehr kompakte Ausgestaltung des gesamten Atemstromsensors ermöglicht.

Bevorzugterweise umfasst der Atemstromsensor zwei Gehäuseteile, nämlich den ersten Strömungsrohrabschnitt und den zweiten Strömungsrohrabschnitt, was einen einfachen Zusammenbau des Atemstromsensors ermöglicht.

Weiter bevorzugt besteht der Atemstromsensor aus Spritzgussteilen oder ist selbst ein Spritzgussteil. Spritzgussteile lassen sich insbesondere bei grösseren Stückzahlen besonders wirtschaftlich herstellen und gegebenenfalls einfach zusammenbauen.

Weiter bevorzugt ist der Atemstromsensor aus einem für medizinische Anwendungen geeigneten Kunststoff hergestellt, womit z. B. auf zusätzliche Beschichtungen im Strömungsrohr verzichtet werden kann.

Vorzugsweise weisen die Verbindungsleitungen jeweils einen weiteren Verbindungsleitungsabschnitt auf, der zumindest bereichsweise gerade verläuft und einen Winkel mit dem im Wesentlichen parallel zur Längsachse des Strömungsrohrs verlaufenden Verbindungsleitungsabschnitt einschliesst, was eine konstruktiv einfache Ausgestaltung der Verbindungsleitung ermöglicht.

Bevorzugt schliesst der zumindest bereichsweise gerade verlaufende, weitere Verbindungsleitungsabschnitt dieser Verbindungsleitung mit dem im Wesentlichen parallel zur Längsachse des Strömungsrohrs verlaufenden Verbindungsleitungsabschnitt dieser Verbindungsleitung einen Winkel von 40° bis 70° ein, so dass eine einfache Fertigung des Atemstromsensors, insbesondere als Spritzgussteil, bei Gewährleitung einer guten Luftführung gegeben ist.

Bevorzugterweise weist der erste Strömungsrohrabschnitt einen im Wesentlichen zylindrischen Abschnitt und einen sich radial erweiternden Abschnitt auf, wobei sich der radial erweiternde Abschnitt zu einem offenen Ende des ersten Strömungsrohrabschnitts hin vergrössert. Damit kann der zweite Strömungsrohrabschnitt einfach an den ersten Strömungsrohrabschnitt angeordnet werden.

Alternativ oder ergänzend weist der zweite Strömungsrohrabschnitt einen im Wesentlichen zylindrischen Abschnitt und einen sich radial erweiternden Abschnitt auf, wobei sich der radial erweiternde Abschnitt zu einem offenen Ende des zweiten Strömungsrohrabschnitts hin vergrössert. Damit kann der erste Strömungsrohrabschnitt einfach an den zweiten Strömungsrohrabschnitt sowie die beiden Strömungsrohrabschnitte dichtend aneinander angeordnet werden.

Insbesondere ist an dem offenen Ende des ersten Strömungsrohrabschnitts sowie des zweiten Strömungsrohrabschnitts jeweils ein radial abragender Flansch mit einer Anlagefläche vorgesehen, so dass der Durchmesser in diesem Bereich grösser ist und somit das Anordnen mithilfe der beiden Anlageflächen der beiden Strömungsrohrabschnitte vereinfacht wird.

Vorzugsweise ist an dem sich radial erweiternden Abschnitt des ersten Strömungsrohrabschnittes eine erste Anschlussausnehmung zur zumindest abschnittsweisen Aufnahme des weiteren Anschlusses des zweiten Strömungsrohrabschnittes vorhanden. Der weitere Anschluss des zweiten Strömungsrohrabschnittes kann somit einfach in dieser ersten Anschlussausnehmung angeordnet werden, sodass die Baugrösse des Atemstromsensors verkleinert werden kann und ein möglichst kompakten Atemstromsensor zur Verfügung gestellt wird.

Insbesondere ist die erste Anschlussausnehmung am radial abragenden Flansch des ersten Strömungsrohrabschnitts angeordnet. Der erste Strömungsrohrabschnitt weist im Bereich des radial abragenden Flansches eine verbesserte Stabilität auf, sodass die erste Anschlussausnehmung ebenfalls eine verbesserte Stabilität bzw. Festigkeit aufweisen kann.

Vorteilhaft ist an dem sich radial erweiternden Abschnitt des ersten Strömungsrohrabschnittes eine erste Stützstruktur zum Abstützen des weiteren Anschlusses des zweiten Strömungsrohrabschnittes, sodass diesem weiteren Anschluss eine vergrösserte Auflagefläche bereitgestellt wird und somit dieser mechanisch stabiler aufgenommen wird und beispielsweise nicht abknicken kann.

Vorteilhaft weist der weitere Anschluss des zweiten Strömungsrohrabschnittes einen Stützabschnitt auf, mit dem dieser Anschluss vorteilhaft an der ersten Anschlussausnehmung an dem sich radial erweiternden Abschnitt des ersten Strömungsrohrabschnittes anliegen kann. Damit wird der weitere Anschluss stabil in dieser Anschlussausnehmung gehalten. Somit ist kann beispielsweise ein Abknicken des weiteren Anschlusses durch externe mechanische Belastungen weiter verhindert werden.

Alternativ oder ergänzend ist an dem sich radial erweiternden Abschnitt des zweiten Strömungsrohrabschnittes eine weitere Anschlussausnehmung zur zumindest abschnittsweisen Aufnahme des ersten Anschlusses des ersten Strömungsrohrabschnittes vorhanden. Der erste Anschluss des ersten Strömungsrohrabschnittes kann somit einfach in dieser zweiten Anschlussausnehmung angeordnet werden, sodass die Baugrösse des Atemstromsensors weiter verkleinert werden kann und ein möglichst kompakter Atemstromsensor zur Verfügung gestellt wird.

Insbesondere ist die zweite Anschlussausnehmung am radial abragenden Flansch des zweiten Strömungsrohrabschnitts angeordnet. Der zweite Strömungsrohrabschnitt weist im Bereich des radial abragenden Flansches eine verbesserte Stabilität auf, sodass die zweite Anschlussausnehmung ebenfalls eine verbesserte Stabilität bzw. Festigkeit aufweisen kann.

Vorteilhaft ist an dem sich radial erweiternden Abschnitt des zweiten Strömungsrohrabschnittes eine weitere Stützstruktur zum Abstützen des ersten Anschlusses des ersten Strömungsrohrabschnittes, sodass dieser Anschluss eine verbesserte mechanische Stabilität aufweist und beispielsweise nicht abknicken kann.

Vorteilhaft weist der erste Anschluss des ersten Strömungsrohrabschnittes einen Stützabschnitt auf, mit dem dieser Anschluss vorteilhaft an der zweiten Anschlussausnehmung an dem sich radial erweiternden Abschnitt des zweiten Strömungsrohrabschnittes anliegen kann. Damit wird der erste Anschluss stabil in dieser Anschlussausnehmung gehalten. Somit kann beispielsweise ein Abknicken des ersten Anschlusses durch externe mechanische Belastungen weiter verhindert werden.

Vorzugsweise schliesst der zumindest bereichsweise gerade verlaufende, weitere Verbindungsleitungsabschnitt der einen Verbindungsleitung mit dem im Wesentlichen parallel zur Längsachse des Strömungsrohrs verlaufenden Verbindungsleitungsabschnitt dieser Verbindungsleitung einen Winkel von 48° bis 62° ein, womit sich zusätzlich zu der einfachen Fertigung des Atemstromsensors, insbesondere als Spritzgussteil, und der Gewährleitung einer guten Luftführung der Nachbearbeitungsaufwand, z. B. beim Entformen des Atemstromsensors, gering ist.

Besonders bevorzugt schliesst der zumindest bereichsweise gerade verlaufende, weitere Verbindungsleitungsabschnitt dieser Verbindungsleitung mit dem im Wesentlichen parallel zur Längsachse des Strömungsrohrs verlaufenden Verbindungsleitungsabschnitt dieser Verbindungsleitung einen Winkel von 52° bis 58° ein, womit der Atomstromsensor neben den zuvor genannten Vorteilen sich hinsichtlich Stabilität sowie Materialbedarf zur Herstellung desselben optimal ausgestalten lässt.

Vorzugsweise schliesst der zumindest bereichsweise gerade verlaufende, weitere Verbindungsleitungsabschnitt der anderen Verbindungsleitung mit dem im Wesentlichen parallel zur Längsachse des Strömungsrohrs verlaufenden Verbindungsleitungsabschnitt der anderen Verbindungsleitung einen Winkel von 130° bis 160° ein, so dass eine einfache Fertigung des Atemstromsensors, insbesondere als Spritzgussteil, bei Gewährleitung einer guten Luftführung gegeben ist.

Vorzugsweise schliesst der zumindest bereichsweise gerade verlaufende, weitere Verbindungsleitungsabschnitt der anderen Verbindungsleitung mit dem im Wesentlichen parallel zur Längsachse des Strömungsrohrs verlaufenden Verbindungsleitungsabschnitt der anderen Verbindungsleitung einen Winkel von 138° bis 152° ein, womit sich zusätzlich zu der einfachen Fertigung des Atemstromsensors, insbesondere als Spritzgussteil, und der Gewährleitung einer guten Luftführung der Nachbearbeitungsaufwand, z.B. beim Entformen des Atemstromsensors, gering ist.

Besonders bevorzugt schliesst der zumindest bereichsweise gerade verlaufende, weitere Verbindungsleitungsabschnitt der anderen Verbindungsleitung mit dem im Wesentlichen parallel zur Längsachse des Strömungsrohrs verlaufenden Verbindungsleitungsabschnitt der anderen Verbindungsleitung einen Winkel von 142° bis 148° ein, womit der Atomstromsensor neben den zuvor genannten Vorteilen sich hinsichtlich Stabilität sowie Materialbedarf zur Herstellung desselben optimal ausgestalten lässt.

Bevorzugt ist der erste Anschluss benachbart zum weiteren Anschluss angeordnet. Damit sind beide Anschlüsse im gleichen Bereich des Strömungsrohres angeordnet, wodurch ein Abknicken der Verbindungsschläuche verhindert wird.

Vorzugsweise ist der erste Anschluss benachbart und beabstandet zum weiteren Anschluss angeordnet, wodurch die Verbindungsschläuche einfach mit den jeweiligen Anschlüssen verbinden lassen.

Vorzugsweise ist der erste Anschluss zumindest abschnittsweise an der äusseren Mantelfläche des ersten Strömungsrohrabschnitts und somit an einer Gehäuseaussenseite des Atemstromsensors angeordnet. Damit lässt sich der Atemstromsensor einfach herstellen und eine absolut dichte Verbindung zwischen dem ersten Anschluss und dem ersten Strömungsrohrabschnitt gewährleisten. Auf eine zusätzliche Nut/Kammstruktur im Bereich des weiteren Verbindungsleitungsabschnitts kann verzichtet werden.

Alternativ oder ergänzend ist der weitere Anschluss zumindest abschnittsweise an der Mantelfläche des zweiten Strömungsrohrabschnitts und somit an einer Gehäuseaussenseite des Atemstromsensors angeordnet. Damit lässt sich eine absolut dichte Verbindung zwischen dem zweiten Anschluss und dem zweiten Strömungsrohrabschnitt gewährleisten. Auf eine zusätzliche Nut/Kammstruktur im Bereich des weiteren Verbindungsleitungsabschnitts kann verzichtet werden. Damit können die beiden Strömungsrohrabschnitte mit einfachen Prozessschritten zusammengeführt werden, wobei der Strömungswiderstand exakt im Strömungsrohr positionierbar ist und die Notwendigkeit der absolut dichten Verbindung zwischen den beiden Strömungsrohrabschnitten gewährleistet ist.

Bevorzugt ist eine Werkzeugöffnung zur Entfernung des Ausformwerkzeugs zur Ausformung des weiteren Verbindungsleitungsabschnitt einer Verbindungsleitung sowie jeweils ein Verschlusselement zum Verschliessen dieser Werkzeugöffnung vorgesehen, wobei das erste Verschlusselement zum Verschliessen des weiteren Verbindungsleitungsabschnitts der ersten Verbindungsleitung vorzugsweise an dem ersten Strömungsrohrabschnitt angeordnet ist und wobei das zweite Verschlusselement zum Verschliessen des weiteren Verbindungsleitungsabschnitts der ersten Verbindungsleitung vorzugsweise an dem zweiten Strömungsrohrabschnitt angeordnet ist, womit der Atemstromsensor einfach herstellbar ist. Mit dem Verschliessen der Werkzeugöffnung wird die Luftführung in der entsprechenden Verbindungsleitung ohne Luftkurzschluss sichergestellt.

Vorzugsweise ist das erste Verschlusselement über ein Scharnier, vorzugsweise über ein Biegescharnier, an dem ersten Strömungsrohrabschnitt verschwenkbar angeordnet. Das zweite Verschlusselement ist bevorzugt ebenfalls über ein Scharnier, vorzugsweise über ein Biegescharnier, an dem zweiten Strömungsrohrabschnitt verschwenkbar angeordnet. Somit sind die Verschlusselemente verliersicher an dem jeweiligen Bauteil angeordnet. Die Verschlusselemente stehen dadurch während dem gesamten Zusammenbau des Atemstromsensors zur Verfügung und können bedarfsweise beziehungsweise im Herstellungsprozess des Atemstromsensors optimiert zum Verschliessen der Werkzeugöffnung angeordnet werden.

Vorteilhaft bestehen die Verschlusselemente aus einem für medizinische Anwendungen geeigneten Material, insbesondere aus einem für medizinische Anwendungen geeigneten Kunststoff. Ist der entsprechende Strömungsrohrabschnitt aus dem gleichen oder einem verbindungstechnisch kompatiblen Material gefertigt, können somit die Werkzeugöffnungen mit einem einfachen Arbeitsschritt geschlossen werden.

Das erste und/oder das zweite Verschlusselement können als Pfropfen ausgebildet sein, der mit einfachen Prozessschritten ein Verschliessen der entsprechenden Werkzeugöffnung ermöglicht.

Bevorzugt sind an den Werkzeugöffnungen und/oder den Verschlusselementen Nut-/Kammstrukturen vorgesehen, womit diese beim Zusammenführen ineinandergreifen und dadurch eine hohe Dichtigkeit des Verschlusses gewährleisten.

Vorzugsweise sind die Verschlusselemente mittels einer dichtend verbindenden Verbindung, besonders bevorzugt mittels einer Ultraschallverschweissung, zum Verschliessen der Werkzeugöffnungen an den jeweiligen Strömungsrohrabschnitt festgelegt, womit eine absolute Dichtigkeit des Verschlusses gewährleistet wird.

In einer alternativen Ausführungsform sind die Verschlusselemente zum Verschliessen der Werkzeugöffnungen an den jeweiligen Strömungsrohrabschnitt mittels einer von der Ultraschallverschweissung verschiedenartigen Verschweissung, mittels eines Passsitzes oder mittels Kleben festgelegt.

In einer weiteren alternativen Ausführungsform wird beispielsweise eine 2-Komponenten-Lösung bei der Herstellung des Atemstromsensors vorgesehen, bei der z. B. im Bereich der Kontaktflächen zumindest eine derselben aus einem weicheren Kunststoff oder einem Kunststoff mit adhäsiven Eigenschaften besteht bzw. beschichtet ist. Beim Zusammenführen der entsprechenden Teile werden diese direkt dichtend miteinander verbunden.

Eine derartige 2-Komponenten-Lösung ist bei der Herstellung des Atemstromsensors für jene Teile geeignet, welche miteinander zu verbinden sind und insbesondere welche dichtend miteinander zu verbinden sind.

Ein erfindungsgemässer Atemadapter umfasst zumindest einen Atemstromsensor, der zumindest einen der vorgenannten Merkmale aufweist, und zumindest einen Verbindungsschlauch, der einen der Anschlüsse des Atemstromsensors mit einer Messeinrichtung verbindet, zur Schaffung einer fluidschen Verbindung von der Messeinrichtung zu dem Atemstromsensor, wobei die fluidische Verbindung direkt in einen der Strömungsrohrabschnitte des Atemstromsensors stossstellenfrei ausgebildet ist.

Vorteilhaft ist die Messeinrichtung zur Messung der Druckdifferenz im Atemstromsensor ausgebildet ist.

Dieser Atemadapter ist einfach in der Anwendung und weist zudem die im Zusammenhang mit dem Atemstromsensor hier genannten Vorteile auf.

Unter dem Begriff "fluidsche Verbindung" wird in diesem Zusammenhang eine für ein Fluid, wie z. B. Luft, durchströmbare und optional dichte Verbindung von einer Eintrittsstelle zu einer Austrittsstelle verstanden.

Vorteilhaft ist an allen Anschlüssen des Atemstromsensors jeweils ein mit der Messeinrichtung verbindbarer Verbindungsschlauch vorgesehen. Um dem Benutzer die Verwendung des Atemadapters zu vereinfachen, können die Verbindungsschläuche optisch, z. B. farblich, und/oder haptisch unterschiedlich ausgebildet werden.

Vorzugsweise ist beim Atemadapter zumindest ein Beatmungsschlauch und ein Mundstück, das dem Patienten zugeführt wird, vorgesehen, wobei der zumindest einen Beatmungsschlauch das Mundstück mit dem zumindest einen Atemstromsensor verbindet, womit ein einfach einsetzbarer Atemadapter geschaffen ist.

Weiter vorteilhaft ist ein weiterer Beatmungsschlauch vorgesehen, welcher ebenfalls mit dem zumindest einen Atemsensor verbunden ist und an einem Beatmungsgerät oder Intensivgerät anschliessbar ist, womit der Benutzer einen einfach einsetzbaren Atemadapter zur Verfügung hat.

Das erfindungsgemässe Verfahren zur Herstellung eines Atemstromsensors ist gekennzeichnet durch die Schritte:
Ausformen des ersten Strömungsrohrabschnitts und des zweiten Strömungsrohrabschnitts jeweils in einer Herstellungswerkzeugform, wobei die Verbindungsleitungsabschnitte und die weiteren Verbindungsleitungsabschnitte der entsprechenden Verbindungsleitungen mittels Schiebern als Ausformwerkzeug und/oder mittels Pins als Ausformwerkzeug ausgebildet werden (Schritt a)).

Alternativ oder ergänzend werden die weiteren Verbindungsleitungsabschnitte der entsprechenden Verbindungsleitungen mittels Pins als Ausformwerkzeug ausgebildet (Schritt a)).

Das Ausformwerkzeug beziehungsweise die Ausformwerkzeuge sind in der jeweiligen Herstellungswerkzeugform angeordnet beziehungsweise in dieser anordnenbar, was eine einfache Herstellung des ersten Strömungsrohrabschnitts wie auch des zweiten Strömungsrohrabschnitts ermöglicht.

Entformen des ersten Strömungsrohrabschnitts und des zweiten Strömungsrohrabschnitts aus der jeweiligen Herstellungswerkzeugform (Schritt b)).

Die als Schieber oder Pins ausgebildeten Ausformwerkzeuge vereinfachen die Entformung des ersten Strömungsrohrabschnitts wie auch des zweiten Strömungsrohrabschnitts aus der jeweiligen Herstellungswerkzeugform massgeblich.

Ausrichten des ersten Strömungsrohrabschnitts und des zweiten Strömungsrohrabschnitts und zwar axial sowie bezüglich der Winkellage zueinander (Schritt c)), womit die korrekte Ausrichtung dieser Teile zueinander und somit die einwandfreie Funktion des Atemstromsensors sichergestellt wird.

Dichtendes Verbinden des ersten Strömungsrohrabschnitts und des zweiten Strömungsrohrabschnitts (Schritt d)). Die dichtend verbindende Verbindung umfasst - als nicht abschliessende Aufzählung - Verschweissungen, insbesondere Ultraschallverschweissungen, Passsitze, Klebeverbindungen oder 2-Komponenten-Lösungen.

Vorteilhaft erfolgt ein dichtendes Verschliessen einer Werkzeugöffnung an den jeweiligen Anschlüssen des Atemstromsensor mithilfe einer Ultraschallverschweissung. Mit dem Verschliessen der Werkzeugöffnung wird die Luftführung in der entsprechenden Verbindungsleitung ohne Luftkurzschluss sichergestellt.

Weitere Vorteile, Merkmale und Einzelheiten der Erfindung ergeben sich aus der nachfolgenden Beschreibung, in der unter Bezugnahme auf die Zeichnungen Ausführungsbeispiele der Erfindung beschrieben sind.

Die Bezugszeichenliste ist wie auch der technische Inhalt der Patentansprüche und Figuren Bestandteil der Offenbarung. Die Figuren werden zusammenhängend und übergreifend beschrieben. Gleiche Bezugszeichen bedeuten entsprechende Bauteile, Bezugszeichen mit unterschiedlichen Indices geben funktionsgleiche oder ähnliche Bauteile an.

Es zeigen dabei:
- Fig. 1: eine erste Ausführungsform eines erfindungsgemässen Atemstromsensors in einer perspektivischen Darstellung;
- Fig. 2: den Atemstromsensor gemäss Fig. 1 in einer perspektivischen Explosionsdarstellung;
- Fig. 3: ein erster Strömungsrohrabschnitt des Atemstromsensors gemäss Fig. 1 in einer Seitenansicht;
- Fig. 4: der erste Strömungsrohrabschnitt gemäss Fig. 3 in einem Längsschnitt;
- Fig. 5: ein Detailausschnitt X der Fig. 4;
- Fig. 6: ein zweiter Strömungsrohrabschnitt des Atemstromsensors gemäss Fig. 1 in einer Seitenansicht;
- Fig. 7: der zweite Strömungsrohrabschnitt gemäss Fig. 6 in einem Längsschnitt;
- Fig. 8: ein Detailausschnitt Y der Fig. 7;
- Fig. 9: einen Atemadapter mit einem Atemstromsensor gemäss den Figuren 1 bis 8 in einer perspektivischen Darstellung;
- Fig. 10: ein erster Strömungsrohrabschnitt eines Atemstromsensors gemäss einer zweiten Ausführungsform in einer Seitenansicht;
- Fig. 11: der erste Strömungsrohrabschnitt gemäss Fig. 10 in einem Längsschnitt;
- Fig. 12: ein zweiter Strömungsrohrabschnitt des Atemstromsensors gemäss der zweiten Ausführungsform in einer Seitenansicht;
- Fig. 13: der zweite Strömungsrohrabschnitt gemäss Fig. 12 in einem Längsschnitt;
- Fig. 14: ein Detailauschnitt Z der Figuren 11 und 13;
- Fig. 15: der erste Strömungsrohrabschnitt eines Atemstromsensors gemäss einer dritten Ausführungsform in einem Längsschnitt;
- Fig. 16: der zweite Strömungsrohrabschnitt des Atemstromsensors gemäss der dritten Ausführungsform in einem Längsschnitt.
- Fig. 17: eine weitere Ausführungsform eines erfindungsgemässen Atemstromsensors in einer perspektivischen Darstellung;
- Fig. 18: der erste Strömungsrohrabschnitt gemäss Fig. 17 in einer perspektivischen Darstellung;
- Fig. 19: der zweite Strömungsrohrabschnitt gemäss Fig. 17 in einer perspektivischen Darstellung;
- Fig. 20: der Atemstromsensor gemäss Fig. 10 in einer weiteren perspektivischen Darstellung;

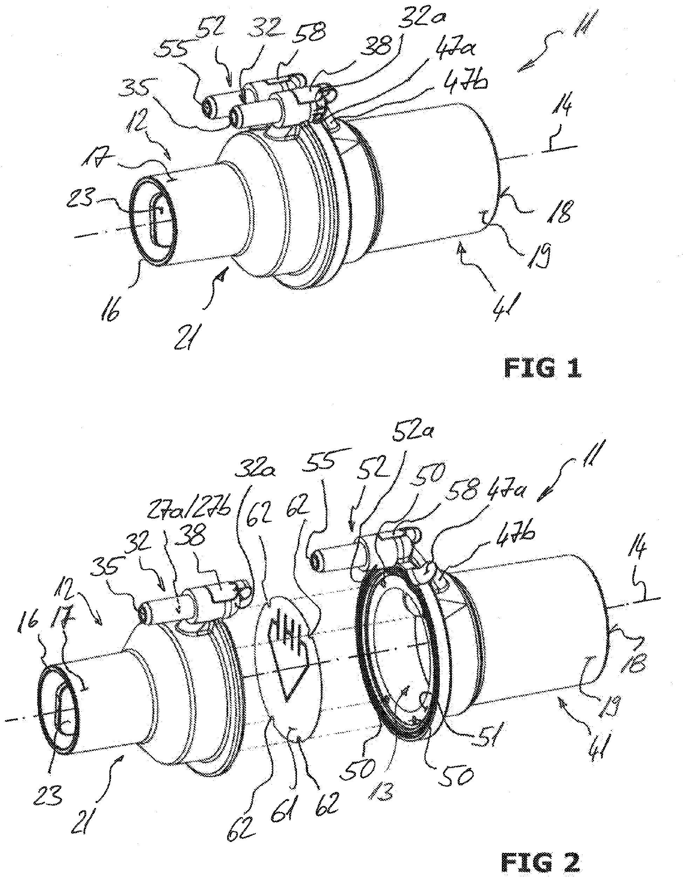

Die in den Figuren 1 bis 8 gezeigte erste Ausführungsform eines Atemstromsensors 11 ist im vorliegenden Beispiel ein Spritzgussteil und der Atemstromsensor 11 ist aus Kunststoff hergestellt.

Wie den Figuren 1 und 2 zeigen weist der Atemstromsensor 11 ein Strömungsrohr 12, das eine Längsachse 14, einen ersten Strömungsrohrabschnitt 21 und einen zweiten Strömungsrohrabschnitt 41 aufweist, sowie im Bereich der beiden freien Enden 16 bzw. 18 jeweils einen Schlauchanschluss 17 bzw. 19 für Beatmungsschläuche aufweist.

Am Schlauchanschluss 17 wird ein Beatmungsschlauch angeordnet, der zu einer, hier nicht dargestellten Mundstück beziehungsweise Beatmungsmaske führt, welche dem Patienten zur Beatmung angelegt wird. Am anderen Schlauchanschluss 19 wird ein Beatmungsschlauch angeordnet, der zu einem Beatmungsgerät führt.

In dem Strömungsrohr 12, das einen Strömungskanal 13 ausbildet, ist zwischen dem ersten Strömungsrohrabschnitt 21 und dem zweiten Strömungsrohrabschnitt 41 ein Strömungswiderstand 61 angeordnet, der als Blendenklappe ausgebildet ist. An dem Strömungswiderstand 61 sind umfänglich Ausnehmungen 62 vorgesehen, welche mit an dem ersten Strömungsrohrabschnitt 21 angeordneten Nocken 30 zusammenwirken und dadurch die richtige Ausrichtung des Strömungswiderstands 61 im Strömungskanal 13 beim Zusammenbau des Atemstromsensors 11 sicherstellen.

Anstelle von vorstehenden Nocken 30 und damit zusammenwirkenden Ausnehmungen 62 kann die korrekte Ausrichtung des Strömungswiderstands 61 im Strömungskanal 13 aber auch die korrekte Ausrichtung des ersten Strömungsrohrabschnitts 21 und des zweiten Strömungsrohrabschnitts 41 über Justiermittel, welche z. B. auch aussenseitig angeordnet sein können, und/oder über eine Nut-/Kammstruktur sichergestellt werden.

Der Atemstromsensor 11 weist zwei Anschlüsse 32 und 52 zur Abnahme der durch den Strömungswiderstand 61 erzeugten Druckdifferenz. Die Öffnungen 35 bzw. 55 der Anschlüsse 32 und 52 sind in die gleiche Richtung, nämlich in Richtung des freien Endes 16 des ersten Strömungsrohrabschnitt 21 ausgerichtet. Dabei sind die Anschlüsse 32 und 52 benachbart und beabstandet zueinander an den äusseren Mantelflächen der jeweiligen Strömungsrohrabschnitten 21 und 41 angeordnet.

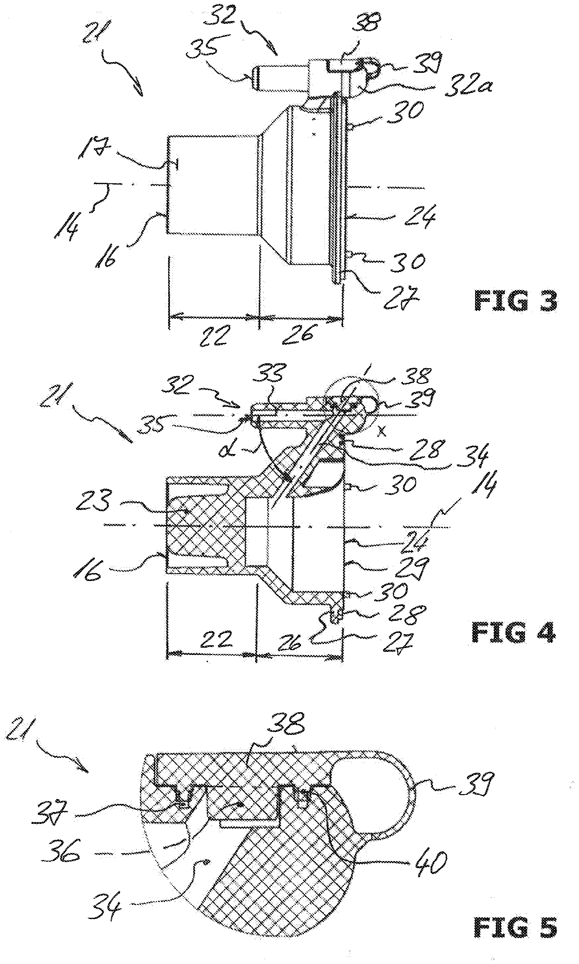

Der erste Strömungsrohrabschnitt 21 (siehe insbesondere auch die Figuren 3 bis 5) ist ein erster Gehäuseteil des Atemstromsensors 11. Der erste Strömungsrohrabschnitt 21 weist einen im Wesentlichen zylindrischen Abschnitt 22 und einen sich radial erweiternden Abschnitt 26 auf, so dass der Durchmesser in diesem Bereich grösser ist. In dem im Wesentlichen zylindrischen Abschnitt 22 ist in dessen Strömungskanalabschnitt ein Leitelement 23 vorgesehen, welches den Strömungskanal 13 in diesem Bereich in zwei gleiche grosse Abschnitte aufteilt und eine gezielte Luftführung im Strömungsrohr 12 ermöglicht.

Im sich radial erweiternden Abschnitt 26 vergrössert sich ebenfalls der entsprechende Strömungskanalabschnitt zum offenen Ende 24 des ersten Strömungsrohrabschnitts 21 hin. An diesem offenen Ende 24 ist ein radial abragender Flansch 27 mit einer Anlagefläche 29 vorgesehen, so dass der Durchmesser in diesem Bereich grösser ist. Die Anlagefläche 29 ist im zusammengeführten Zustand des Atemstromsensors 11 dem zweiten Strömungsrohrabschnitt 41 zugewandt. An diesem Flansch 27 ist auf der Anlagefläche 29 ein erster umlaufender Teil 28 einer Nut-/Kammstruktur vorgesehen. An dem sich radial erweiternden Abschnitt 26 ist im Bereich des Flansches 27 eine erste Anschlussausnehmung 27a vorhanden, welche von einer ersten Stützstruktur 27b gestützt wird. Der weitere Anschluss 52 des zweiten Strömungsrohrabschnittes 41 weist benachbart zur Öffnung 55 einen Stützabschnitt 52a auf, welche im zusammengefügten Zustand in der ersten Anschlussausnehmung 27a des Atemstromsensors 11 anliegt bzw. aufliegt und von der ersten Stützstruktur 27b gestützt wird.

Von der Anlagefläche 29 ragen die Nocken 30 zur korrekten Ausrichtung des Strömungswiderstands 61 im Strömungsrohr 12 beim Zusammenbau des Atemstromsensors 11 ab.

Der erste Anschluss 32 zur Abnahme der durch den Strömungswiderstand 61 erzeugten Druckdifferenz mündet über eine erste Verbindungsleitung in den Strömungskanalabschnitt des ersten Strömungsrohrabschnitts 21. Die erste Verbindungsleitung weist einen Verbindungsleitungsabschnitt 33, der im Wesentlichen parallel zur Längsachse 14 des Strömungsrohrs 12 verläuft, sowie einen weiteren Verbindungsleitungsabschnitt 34 auf, der bereichsweise gerade verläuft und einen Winkel α von 40° bis 70°, vorzugsweise von 48° bis 62° und besonders bevorzugt von 52° bis 58°, mit dem im Wesentlichen parallel zur Längsachse 14 des Strömungsrohrs 12 verlaufenden Verbindungsleitungsabschnitt 33 einschliesst. Im vorliegenden Beispiel beträgt dieser Winkel α 55°.

In Verlängerung des weiteren Verbindungsleitungsabschnitts 34 der ersten Verbindungsleitung ist an dem ersten Strömungsrohrabschnitt 21 eine erste Werkzeugöffnung 36 zur Entfernung des Ausformwerkzeugs zur Ausformung des weiteren Verbindungsleitungsabschnitt 34 sowie ein erstes Verschlusselement 38 zum Verschliessen dieser Werkzeugöffnung 36 vorgesehen. Das erste Verschlusselement 38 ist über ein Scharnier, hier über ein Biegescharnier 39 oder ein Filmscharnier, an dem ersten Strömungsrohrabschnitt 21 verschwenkbar angeordnet.

Um die erste Werkzeugöffnung 36 herum ist eine Nutstruktur 37 ausgebildet. An dem ersten Verschlusselement 38 ist eine Kammstruktur 40 ausgebildet, welche beim Verschliessen der ersten Werkzeugöffnung 36 in die Nutstruktur 37 eingreift und bei einem Verschweissvorgang, insbesondere bei einem Ultraschweissvorgang, einen dichten Verschluss der ersten Werkzeugöffnung 36 und somit die Dichtigkeit der ersten Verbindungsleitung sicherstellt.

In der Darstellung gemäss der Figur 5 ist die erste Werkzeugöffnung 36 im verschlossenen Zustand gezeigt.

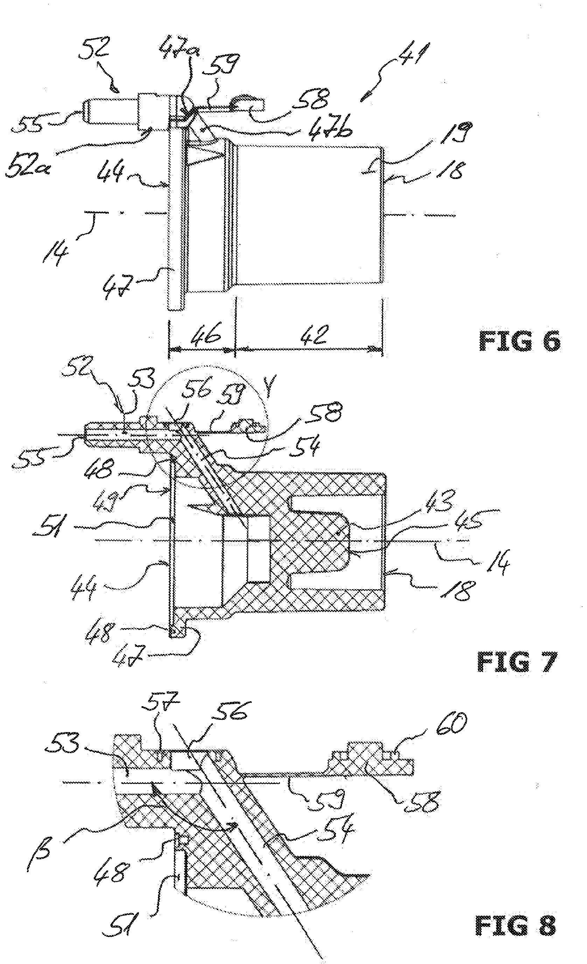

Der zweite Strömungsrohrabschnitt 41 (siehe insbesondere auch die Figuren 6 bis 8) ist ein zweiter Gehäuseteil des Atemstromsensors 11. Der zweite Strömungsrohrabschnitt 41 weist einen im Wesentlichen zylindrischen Abschnitt 42, der sich aussenseitig in Richtung des freien Endes 18 verjüngt, und einen radial vergrösserten Abschnitt 46 auf. In dem im Wesentlichen zylindrischen Abschnitt 42 ist in dessen Strömungskanalabschnitt ein Leitelement 43 vorgesehen, welches das Strömungsrohr 12 in diesen Bereich in zwei gleiche grosse Abschnitte aufteilt und eine gezielte Luftführung im Strömungsrohr 12 ermöglicht. Das freie Ende 45 des Leitelement 43 ist in Bezug auf das freie Ende 18 des Strömungsrohrs 12 zurückversetzt.

Im radial, d. h. nach aussen, vergrösserten Abschnitt 46 vergrössert sich ebenfalls der entsprechende Strömungskanalabschnitt zum offenen Ende 44 des zweiten Strömungsrohrabschnitts 41 hin. An diesem offenen Ende 44 ist ein radial abragender Flansch 47 mit einer Anlagefläche 49 vorgesehen, welche im zusammengeführten Zustand des Atemstromsensors 11 dem ersten Strömungsrohrabschnitt 21 beziehungsweise dessen Anlagefläche 29 zugewandt ist. An diesem Flansch 47 ist auf der Anlagefläche 49 ein zweiter umlaufender Teil 48 einer Nut-/Kammstruktur vorgesehen, der in den ersten umlaufenden Teil 28 der Nut-/Kammstruktur eingreift und beim Verschweissen, insbesondere beim Ultraschall-Verschweissen, mit diesem verschmilzt. Damit wird eine dichte Verbindung des ersten Strömungsrohrabschnitts 21 mit dem zweiten Strömungsrohrabschnitt 41 in einfacher Form gewährleistet. Eine komplexe, Mehrfachstruktur für die Nut-/Kammstruktur oder Druckflächen, wie es in der CH 701 755 B1 vorgeschlagen ist, erübrigt sich bei der erfindungsgemässen Lösung.

Versetzt zu der Anlagefläche 49 ist am Flansch 47 eine Aufnahmevertiefung 51 vorgesehen, in welcher der Strömungswiderstand 61 zumindest bereichsweise aufgenommen wird. An dem kreiszylindrischen Ring der Aufnahmevertiefung 51 sind Löcher 50 angeordnet, in welche die Nocken 30 des ersten Strömungsrohrabschnitts 21 beim Zusammenbau des Atemstromsensors 11 eindringen. Diese Ausgestaltung ermöglicht nicht nur die korrekte Ausrichtung des Strömungswiderstands 61 im Strömungskanal 13 beim Zusammenbau des Atemstromsensors 11, sondern in Kombination mit den Nut-/Kammstrukturen 28 bzw. 48 an den Flanschen 27 bzw. 47 auch die Anordnung von Strömungswiderständen von unterschiedlicher Dicke, je nach Einsatzgebiet oder Bedarf. Damit ist der Atemstromsensor 11 flexibel einsetzbar und kann ohne grossen Aufwand an andere Typen von Strömungswiderständen 61 angepasst werden. Der verwendete Strömungswiderstand 61 ist zwischen dem ersten Strömungsrohrabschnitt 21 und dem zweiten Strömungsrohrabschnitt 41 gehalten, insbesondere eingeklemmt oder anderweitig gehalten, wie bereichsweise oder vollständig eingeklebt.

An den Flanschen 27 und 47 bilden sich im zusammengebauten Zustand des Atemstromsensors 11 keine Flanschdruckflächen aus. Die Verbindung zwischen dem ersten Strömungsrohrabschnitt 21 als erster Gehäuseteil des Atemstromsensors 11 und dem zweiten Strömungsrohrabschnitt 41 als zweiter Gehäuseteil des Atemstromsensors 11 erfolgt lediglich durch die einfach gestalteten Nut-/Kammstrukturen 28 bzw. 48 an den Flanschen 27 bzw. 47.

An dem sich radial erweiternden Abschnitt 46 ist im Bereich des Flansches 47 eine weitere Anschlussausnehmung 47a vorhanden, welche von einer weiteren Stützstruktur 47b gestützt wird. Der erste Anschluss 32 des ersten Strömungsrohrabschnittes 21 weist benachbart zum ersten Verschlusselement 38 einen Stützabschnitt 32a auf, welcher im zusammengefügten Zustand in der weiteren Anschlussausnehmung 47a des Atemstromsensors 11 anliegt bzw. aufliegt und von der Stützstruktur 47b gestützt wird.

Der zweite Anschluss 52 zur Abnahme der durch den Strömungswiderstand 61 erzeugten Druckdifferenz mündet über eine zweite Verbindungsleitung in den Strömungskanalabschnitt des zweiten Strömungsrohrabschnitts 41. Die zweite Verbindungsleitung weist einen Verbindungsleitungsabschnitt 53, der im Wesentlichen parallel zur Längsachse 14 des Strömungskanals 13 verläuft, sowie einen weiteren Verbindungsleitungsabschnitt 54 auf, der bereichsweise gerade verläuft und einen Winkel β von 130° bis 160°, vorzugsweise von 138° bis 152° und besonders bevorzugt von 142° bis 148°, einschliesst. Im vorliegenden Beispiel beträgt dieser Winkel β 145°.

In Verlängerung des weiteren Verbindungsleitungsabschnitts 54 der zweiten Verbindungsleitung ist an dem zweiten Strömungsrohrabschnitt 41 eine zweite Werkzeugöffnung 56 zur Entfernung des Ausformwerkzeugs zur Ausformung des weiteren Verbindungsleitungsabschnitt 54 sowie ein zweites Verschlusselement 58 zum Verschliessen dieser Werkzeugöffnung 56 vorgesehen. Das zweite Verschlusselement 58 ist über ein Scharnier, hier über ein Biegescharnier 59 oder ein Filmscharnier, an dem zweiten Strömungsrohrabschnitt 41 verschwenkbar angeordnet.

Um die zweite Werkzeugöffnung 56 herum ist eine Nutstruktur 57 ausgebildet. An dem zweiten Verschlusselement 58 ist eine Kammstruktur 60 ausgebildet, welche beim Verschliessen der ersten Werkzeugöffnung 36 in die Nutstruktur 57 eingreift und bei einem Verschweissvorgang, insbesondere bei einem Ultraschweissvorgang, einen dichten Verschluss der zweiten Werkzeugöffnung 56 und somit die Dichtigkeit der zweiten Verbindungsleitung sicherstellt.

In der Darstellung gemäss der Figur 8 ist die zweite Werkzeugöffnung 56 im offenen Zustand gezeigt.

Zur Herstellung eines Atemstromsensors 11 wird in einer Herstellungswerkzeugform der erste Strömungsrohrabschnitt 21 ausgeformt, wobei der Verbindungsleitungsabschnitt 33 mittels eines Pins als Ausformwerkzeug ausgebildet wird und der weitere Verbindungsleitungsabschnitt 34 mittels eines Schiebers als Ausformwerkzeug ausgebildet wird. Anschliessend wird der erste Strömungsrohrabschnitt 21 aus der Herstellungswerkzeugform entformt, wobei dafür zuvor der Schieber aus dem ersten Strömungsrohrabschnitt 21 herausgezogen wird.

Gleichzeitig oder zeitversetzt in einer weiteren Herstellungswerkzeugform der zweite Strömungsrohrabschnitt 41 ausgeformt, wobei der Verbindungsleitungsabschnitt 53 mittels eines Pins als Ausformwerkzeug ausgebildet wird und der weitere Verbindungsleitungsabschnitt 54 mittels eines Schiebers als Ausformwerkzeug ausgebildet wird. Anschliessend wird der zweite Strömungsrohrabschnitt 41 aus der Herstellungswerkzeugform entformt, wobei dafür zuvor der Schieber aus dem zweiten Strömungsrohrabschnitt 41 herausgezogen wird.

Die Werkzeugöffnungen 36 bzw. 56 können nun bereits mit dem Verschlusselement 38 bzw. 58 verschlossen werden.

Nun wird der erste Strömungsrohrabschnitt 21 und der zweite Strömungsrohrabschnitt 41 zueinander ausgerichtet und zwar axial sowie bezüglich der Winkellage zueinander.

Dann werden der erste Strömungsrohrabschnitt 21 und der zweite Strömungsrohrabschnitt 41 dichtend miteinander verbunden, im vorliegenden Ausführungsbeispiel durch eine Ultraschallverschweissung.

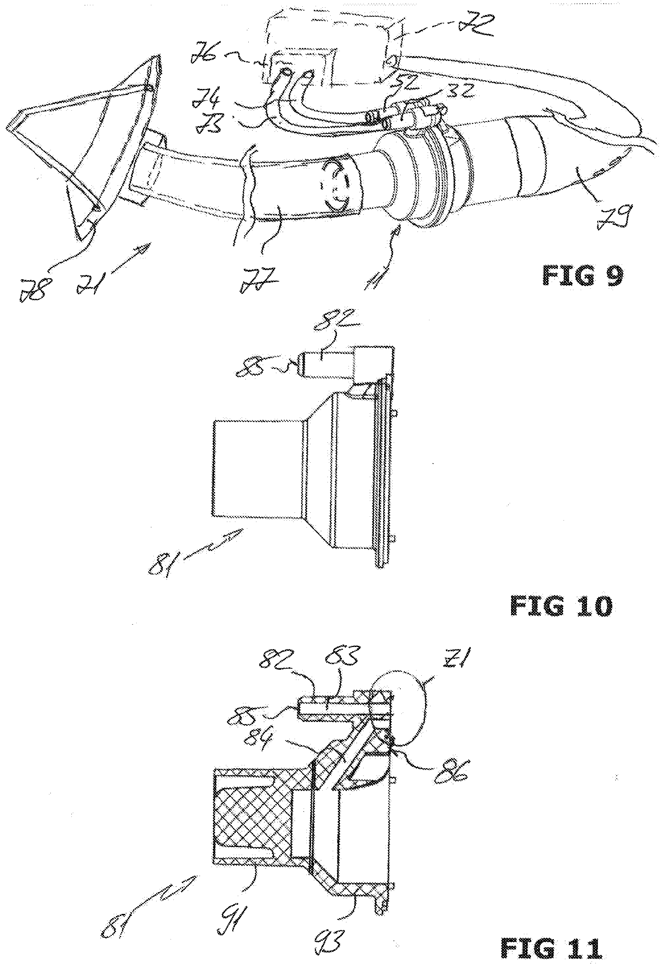

Der in der Figur 9 gezeigte Atemadapter 71 umfasst einen Atemstromsensor 11, einen Verbindungsschlauch 73, der den Anschluss 32 des Atemstromsensors 11 mit einer Messeinrichtung 76 verbindet, zur Schaffung einer ersten fluidschen Verbindung von der Messeinrichtung 76 zu dem Atemstromsensor 11, einen Verbindungsschlauch 74, der den Anschluss 52 des Atemstromsensors 11 mit einer Messeinrichtung 76 verbindet, zur Schaffung einer zweiten fluidschen Verbindung von der Messeinrichtung 76 zu dem Atemstromsensor 11. Die fluidische Verbindungen sind direkt in einen der Strömungsrohrabschnitte des Atemstromsensors 11 mündend stossstellenfrei ausgebildet.

Weiter umfasst der Atemadapter 71 einen ersten Beatmungsschlauch 77 und ein Mundstück 78, das als Beatmungsmaske ausgebildet ist. Der erste Beatmungsschlauch 77 verbindet das Mundstück 78 mit dem Atemstromsensor 11. Zudem weist der Atemadapter 71 einen zweiten Beatmungsschlauch 79 auf, welcher den Atemstromsensor 11 mit einem Beatmungsgerät 72 verbindet.

Das Messgerät 76 ist zur Messung beziehungsweise Erfassung sowie Verarbeitung der im Atemstromsensor 11 erzeugten Druckdifferenz ausgebildet. Im vorliegenden Beispiel ist das Messgerät 76 in dem Beatmungsgerät 72 integriert, das zur künstlichen Beatmung eines Patienten ausgebildet ist.

Bei dem in den Figuren 10 bis 14 dargestellten zweiten Ausführungsbeispiel eines Atemstromsensors unterscheidet sich der erste Strömungsrohrabschnitt 81 (siehe insbesondere die Figuren 10 und 11) von dem ersten Strömungsrohrabschnitt 21 des Atemstromsensors 11 im Wesentlichen nur dadurch, dass dieser mehrteilig ausgebildet ist und eine Verbindungsleitung aufweist, wobei der von der Öffnung 85 des Anschlusses 82 ausgehende Verbindungsleitungsabschnitt 83 in keiner direkten Verbindung mit dem weiteren Verbindungsleitungsabschnitt 84 steht. Der Verbindungsleitungsabschnitt 83 wie auch der weitere Verbindungsleitungsabschnitt 84 münden jeweils zum offenen Ende 86 des ersten Strömungsrohrabschnitts 81. Der Anschluss 82 ist weiterhin an dem des ersten Strömungsrohrabschnitts 81 angeordnet.

Der erste Strömungsrohrabschnitt 81 ist zweiteilig ausgebildet, nämlich Strömungsrohrabschnittsteil 91 und Strömungsrohrabschnittsteil 93, welche mittels einer dichtend verbindenden Verbindung miteinander verbunden sind. Infolge der Zweiteiligkeit des ersten Strömungsrohrabschnitts 81 kann die Struktur der Verbindungsleitung in diesem in der gewünschten Art und Weise bei Gewährleistung einer einfachen Herstellung ausgeführt werden. Eine Herstellungs- beziehungsweise Werkzeugöffnung ist damit zur Ausformung des ersten Strömungsrohrabschnitts 81 nicht erforderlich.

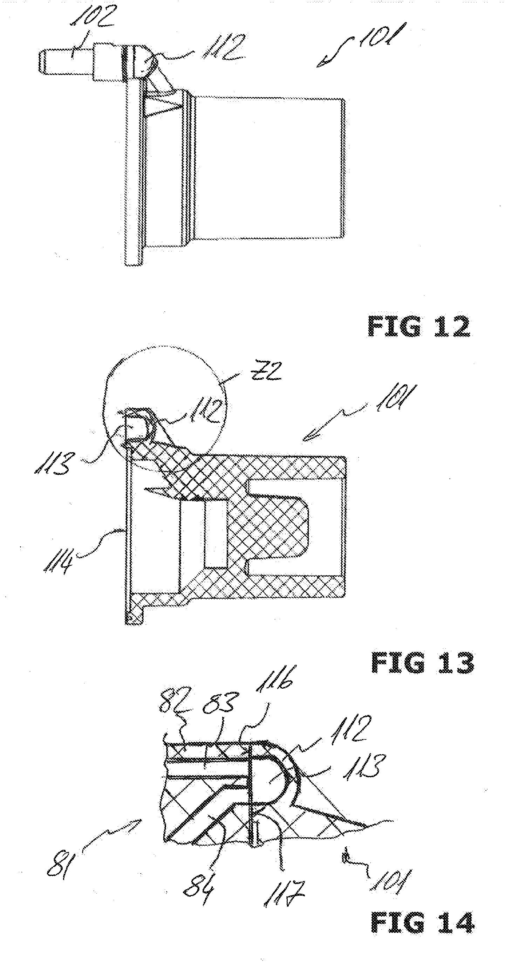

Der zweite Strömungsrohrabschnitt 101 der zweiten Ausführungsform eines Atemstromsensors (siehe insbesondere die Figuren 12 und 13) entspricht in der Ausgestaltung im Wesentlichen dem zweiten Strömungsrohrabschnitt 41 des Atemstromsensors 11. Dieser zweite Strömungsrohrabschnitt 101 weist jedoch im Bereich, welcher im zusammengefügten Zustand dem Anschluss 82 des ersten Strömungsrohrabschnitts 81 gegenüberliegt, eine Luftumlenkung 112 auf, dessen Hohlraum 113 zum freien Ende 114 des zweiten Strömungsrohrabschnitts 101 offen ist.

Im zusammengefügten Zustand des ersten Strömungsrohrabschnitts 81 und des zweiten Strömungsrohrabschnitts 101, wie er in der Figur 14 im Detail dargestellt ist, lässt sich der Verlauf der ersten Verbindungsleitung vom Anschluss 82 in das Innere des erste Strömungsrohrabschnitts 81 erkennen. Das Fluid wird im Hohlraum 113 umgeleitet. Die dichtende Verbindung zwischen dem ersten Strömungsrohrabschnitt 81 und dem zweiten Strömungsrohrabschnitt 101 im Bereich dieser Umlenkung ist nur in den Kontaktabschnitten 116 und 117 gegeben. Bei dieser Ausführungsform ist die erste Verbindungsleitung nicht stossstellenfrei ausgebildet. Jedoch ist die zweite Verbindungsleitung im zweiten Strömungsrohrabschnitt 101 weiterhin stossstellenfrei.

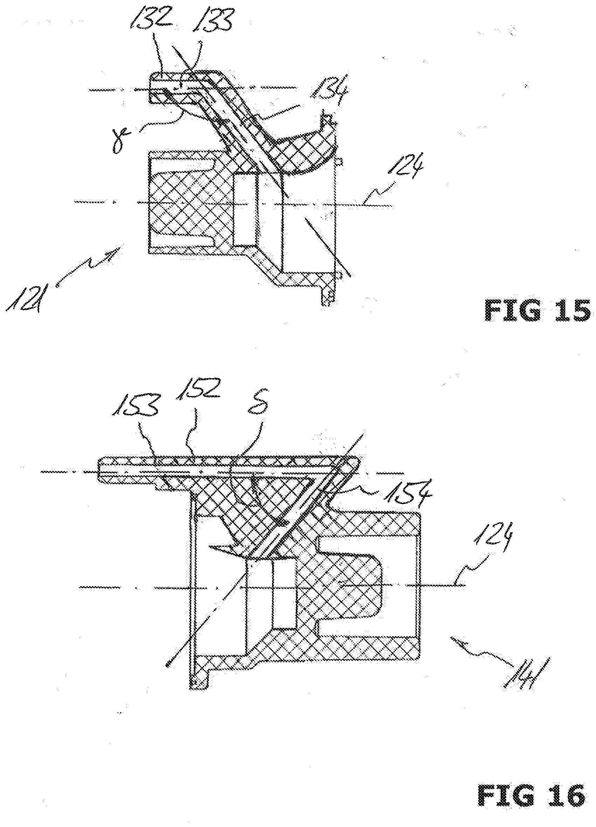

Der erste Strömungsrohrabschnitt 121 (siehe Figur 15) und der zweite Strömungsrohrabschnitt 141 (siehe Figur 16) eines Atemstromsensors gemäss einer dritten Ausführungsform unterscheiden sich nur durch die Ausbildung deren Verbindungsleitungen.

Der erste Anschluss 132 zur Abnahme der durch den Strömungswiderstand erzeugten Druckdifferenz mündet über eine erste Verbindungsleitung in den Strömungskanalabschnitt des ersten Strömungsrohrabschnitts 121. Der im Wesentlichen parallel zur Längsachse 124 des Strömungsrohrs verlaufende Verbindungsleitungsabschnitt 133 und der weitere Verbindungsleitungsabschnitt 134 schliessen einen Winkel γ von 130° bis 160°, vorzugsweise von 138° bis 152° und besonders bevorzugt von 142° bis 148°, ein. Im vorliegenden Beispiel beträgt dieser Winkel γ etwa 147°. Dieser Winkel γ wurde so gewählt, damit das als Schieber ausgebildete Ausformwerkzeug beim Entformen des ersten Strömungsrohrabschnitts 121 einfach herausgezogen werden kann, ohne dass dafür eine Werkzeugöffnung erforderlich ist.

Der zweite Anschluss 152 zur Abnahme der durch den Strömungswiderstand erzeugten Druckdifferenz mündet über eine zweite Verbindungsleitung in den Strömungskanalabschnitt des zweiten Strömungsrohrabschnitts 141. Der im Wesentlichen parallel zur Längsachse 124 des Strömungsrohrs verlaufende Verbindungsleitungsabschnitt 153 und der weitere Verbindungsleitungsabschnitt 154 schliessen einen Winkel δ von 40° bis 70°, vorzugsweise von 48° bis 62° und besonders bevorzugt von 52° bis 58°, mit dem im Wesentlichen parallel zur Längsachse 124 des Strömungsrohrs verlaufenden Verbindungsleitungsabschnitt 153 ein. Im vorliegenden Beispiel beträgt dieser Winkel δ 53°. Dieser Winkel δ wurde so gewählt, damit das als Schieber ausgebildete Ausformwerkzeug beim Entformen des zweiten Strömungsrohrabschnitts 141 einfach herausgezogen werden kann, ohne dass dafür eine Werkzeugöffnung erforderlich ist.

Die erste Verbindungsleitung und die zweite Verbindungsleitung sind jeweils stossstellenfrei ausgebildet.

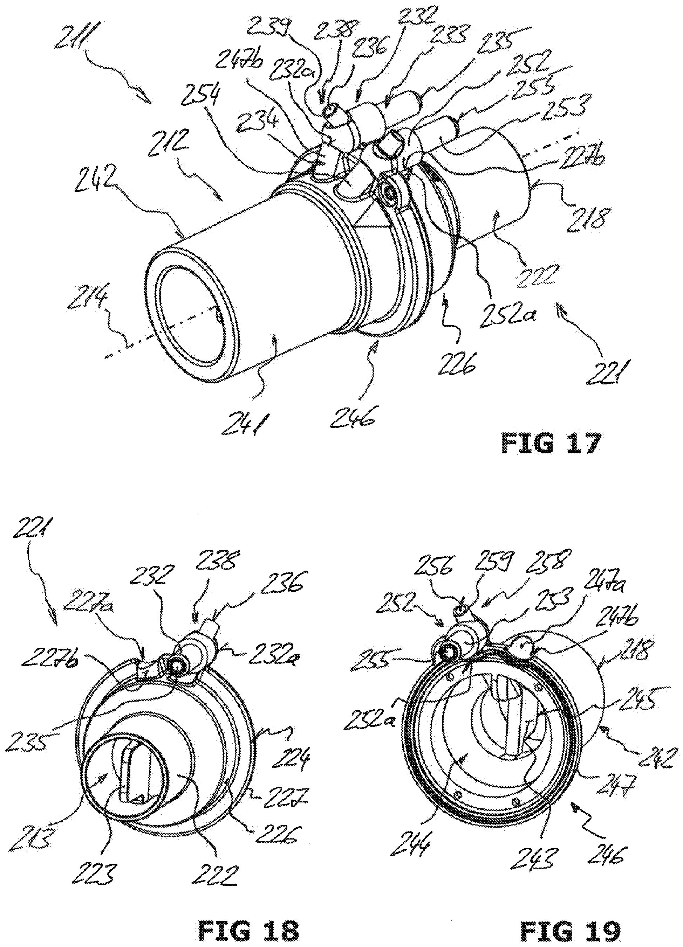

Bei dem in den Figuren 17 bis Figur 20 dargestellten weiteren Ausführungsbeispiel eines Atemstromsensors 211 weist ein Strömungsrohr 212, eine Längsachse 214, einen ersten Strömungsrohrabschnitt 221 und einen zweiten Strömungsrohrabschnitt 241 auf, wobei sich der erste Strömungsrohrabschnitt 221 (siehe insbesondere die Figur 18) von dem ersten Strömungsrohrabschnitt 21 des Atemstromsensors 11 im Wesentlichen nur dadurch unterscheidet, dass ein erstes Verschlusselement 238 am Anschluss 232 als Rohrabschnitt 239 ausgebildet ist. Das erste Verschlusselement 238 weist eine erste Werkzeugöffnung 236 auf, welche in den weiteren Verbindungsleitungsabschnitt 234 mündet, wobei der von der Öffnung 235 des Anschlusses 232 ausgehende Verbindungsleitungsabschnitt 233 in direkter Verbindung mit dem weiteren Verbindungsleitungsabschnitt 234 steht. Der erste Anschluss 232 ist weiterhin an dem des ersten Strömungsrohrabschnitts 221 angeordnet.

Der erste Strömungsrohrabschnitt 221 ist ein erster Gehäuseteil des Atemstromsensors 211. Der erste Strömungsrohrabschnitt 221 weist einen im Wesentlichen zylindrischen Abschnitt 222 und einen sich radial erweiternden Abschnitt 226 auf, so dass der Durchmesser in diesem Bereich grösser ist. In dem im Wesentlichen zylindrischen Abschnitt 222 ist in dessen Strömungskanalabschnitt ein Leitelement 223 vorgesehen, welches den Strömungskanal 213 in diesem Bereich in zwei gleiche grosse Abschnitte aufteilt und eine gezielte Luftführung im Strömungsrohr 212 ermöglicht.

Im sich radial erweiternden Abschnitt 226 des ersten Strömungsrohrabschnitts 221 vergrössert sich ebenfalls der entsprechende Strömungskanalabschnitt zum offenen Ende 224 des ersten Strömungsrohrabschnitts 221 hin (siehe insbesondere Figur 18). An diesem offenen Ende 224 ist ein radial abragender Flansch 227 vorgesehen. An dem sich radial erweiternden Abschnitt 226 ist im Bereich des Flansches 227 eine erste Anschlussausnehmung 227a vorhanden, welche von einer ersten Stützstruktur 227b gestützt wird. Der weitere Anschluss 252 des zweiten Strömungsrohrabschnittes 241 weist benachbart zur Öffnung 255 einen Stützabschnitt 252a auf, welcher im zusammengefügten Zustand in der ersten Anschlussausnehmung 227a des Atemstromsensors 11 anliegt bzw. aufliegt.

Der zweite Strömungsrohrabschnitt 241 dieses Ausführungsbeispiels des Atemstromsensors 211 (siehe insbesondere die Figur 19) entspricht in der Ausgestaltung im Wesentlichen dem zweiten Strömungsrohrabschnitt 41 des Atemstromsensors 11. Dieser zweite Strömungsrohrabschnitt 241 weist jedoch ein Verschlusselement 258 am zweiten Anschluss 252 auf, welches als Rohrabschnitt 259 ausgebildet ist. Das Verschlusselement 258 weist eine zweite Werkzeugöffnung 256 auf, welche in den weiterer Verbindungsleitungsabschnitt 254 mündet, wobei der von der Öffnung 255 des zweiten Anschlusses 252 ausgehende Verbindungsleitungsabschnitt 253 in direkter Verbindung mit dem weiteren Verbindungsleitungsabschnitt 254 steht. Der zweite Anschluss 252 ist weiterhin an dem des zweiten Strömungsrohrabschnitts 241 angeordnet.

Der zweite Strömungsrohrabschnitt 241 ist weiterhin ein zweiter Gehäuseteil des Atemstromsensors 211. Der zweite Strömungsrohrabschnitt 241 weist einen im Wesentlichen zylindrischen Abschnitt 242 auf, der sich aussenseitig in Richtung des freien Endes 218 verjüngt. Der zweite Strömungsrohrabschnitt 241 weist einen zum offenen Ende 244 radial vergrösserten Abschnitt 246 auf. In dem im Wesentlichen zylindrischen Abschnitt 242 ist in dessen Strömungskanalabschnitt ein Leitelement 243 vorgesehen, welches das Strömungsrohr 212 in diesen Bereich in zwei gleiche grosse Abschnitte aufteilt und eine gezielte Luftführung im Strömungsrohr 212 ermöglicht. Das freie Ende 245 des Leitelement 243 ist in Bezug auf das freie Ende 218 des Strömungsrohrs 212 zurückversetzt.

An dem sich radial erweiternden Abschnitt 246 des zweiten Strömungsrohrabschnitts 241 ist im Bereich des Flansches 247 eine weitere Anschlussausnehmung 247a vorhanden, welche von einer weiteren Stützstruktur 247b gestützt wird. Der erste Anschluss 232 des ersten Strömungsrohrabschnittes 221 weist benachbart zum ersten Verschlusselement 238 einen Stützabschnitt 232a auf, welche im zusammengefügten Zustand in der weiteren Anschlussausnehmung 247a des Atemstromsensors 11 anliegt bzw. aufliegt.

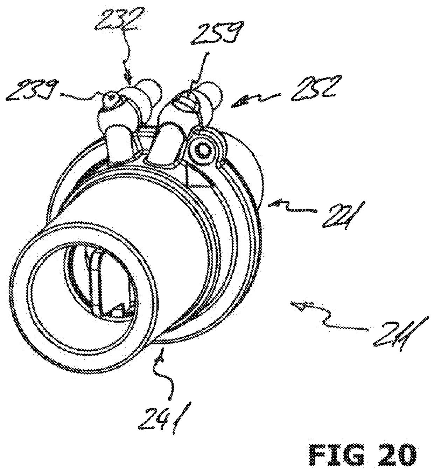

Fig. 20 zeigt den zusammengefügten Atemstromsensor 211, wobei die Werkzeugöffnungen 236 bzw. 256 an den Rohrabschnitten 239 bzw. 259 verschlossen dargestellt sind. Diese wurden in einem Ultraschweissvorgang mit einer Ultraschweisseinrichtung gasdicht verschlossen.

### Bezugszeichenliste

- 11: Atemstromsensor
- 12: Strömungsrohr
- 13: Strömungskanal
- 14: Längsachse v. 12

- 16: freies Ende v. 12
- 17: Schlauchanschluss
- 18: freies Ende v. 12
- 19: Schlauchanschluss

- 21: 1. Strömungsrohrabschnitt
- 22: zylindrischer Abschnitt
- 23: Leitelement
- 24: offenes Ende v. 21

- 26: erweitender Abschnitt
- 27: Flansch
- 27a: 1. Anschlussausnehmung
- 27b: 1. Stützstruktur
- 28: 1. umlaufender Teil einer Nut-/Kammstruktur
- 29: Anlagefläche
- 30: Nocke

- 32: 1. Anschluss
- 32a: Stützabschnitt v. 32
- 33: Verbindungsleitungsabschnitt
- 34: weiterer Verbindungsleitungsabschnitt
- 35: Öffnung v. 32
- 36: 1. Werkzeugöffnung
- 37: Nutstruktur
- 38: 1. Verschlusselement
- 39: Biegescharnier
- 40: Kammstruktur
- 41: 2. Strömungsrohrabschnitt
- 42: zylindrischer Abschnitt
- 43: Leitelement
- 44: offenes Ende v. 21
- 45: freies Ende v. 43
- 46: vergrösserten Abschnitt
- 47: Flansch
- 47a: weitere Anschlussausnehmung
- 47b: weitere Stützstruktur
- 48: 2. umlaufender Teil einer Nut-/Kammstruktur
- 49: Anlagefläche
- 50: Loch
- 51: Aufnahmevertiefung
- 52: 2. Anschluss
- 52a: Stützabschnitt v. 52
- 53: Verbindungsleitungsabschnitt
- 54: weiterer Verbindungsleitungsabschnitt
- 55: Öffnung v. 32
- 56: 2. Werkzeugöffnung
- 57: Nutstruktur
- 58: 2. Verschlusselement
- 59: Biegescharnier
- 60: Kammstruktur
- 61: Strömungswiderstand
- 62: Ausnehmungen von 61

- α: Winkel zw. 33 und 34
- β: Winkel zw. 53 und 54

- 71: Atemadapter
- 72: Beatmungsgerät
- 73: 1. Verbindungsschlauch
- 74: 2. Verbindungsschlauch

- 76: Messgerät/Beatmungsgerät
- 77: 1. Beatmungsschlauch
- 78: Mundstück
- 79: 2. Beatmungsschlauch
- 81: 1. Strömungsrohrabschnitt
- 82: 1. Anschluss
- 83: Verbindungsleitungsabschnitt
- 84: weiterer Verbindungsleitungsabschnitt
- 85: Öffnung v. 82
- 86: offenes Ende v. 81

- 91: 1. Strömungsrohrabschnittsteil

- 93: 2. Strömungsrohrabschnittsteil

- 101: 2. Strömungsrohrabschnitt
- 102: 2. Anschluss

- 112: Luftumlenkung
- 113: Hohlraum v. 112
- 114: freies Ende v. 101

- 116: Kontaktabschnitt
- 117: Kontaktabschnitt

- 121: 1. Strömungsrohrabschnitt

- 124: Längsachse

- 132: 1. Anschluss
- 133: Verbindungsleitungsabschnitt
- 134: weiterer Verbindungsleitungsabschnitt

- 141: 2. Strömungsrohrabschnitt

- 152: 2. Anschluss
- 153: Verbindungsleitungsabschnitt
- 154: weiterer Verbindungsleitungsabschnitt
- 211: Atemstromsensor
- 212: Strömungsrohr
- 213: Strömungskanal
- 214: Längsachse
- 218: freies Ende v. 212
- 221: 1. Strömungsrohrabschnitt
- 222: zylindrischer Abschnitt
- 223: Leitelement
- 224: offenes Ende v. 212
- 226: erweiternder Abschnitt
- 227: Flansch
- 227a: 1. Anschlussausnehmung
- 227b: 1. Stützstruktur

- 232: 1. Anschluss
- 232a: Stützabschnitt v. 232
- 233: Verbindungsleitungsabschnitt
- 234: weiterer Verbindungsleitungsabschnitt
- 235: Öffnung
- 236: 1. Werkzeugöffnung
- 238: 1. Verschlusselement
- 239: Rohrabschnitt

- 241: 2. Strömungsrohrabschnitt
- 242: zylindrischen Abschnitt
- 243: Leitelement
- 244: offenen Ende
- 245: freies Ende v. 241
- 246: erweiternder Abschnitt
- 247: Flansch
- 247a: weitere Anschlussausnehmung
- 247b: weiteren Stützstruktur

- 252: 2. Anschluss
- 252a: Stützabschnitt v. 252
- 253: Verbindungsleitungsabschnitt
- 254: weiterer Verbindungsleitungsabschnitt
- 255: Öffnung
- 256: 2. Werkzeugöffnung
- 258: Verschlusselement
- 259: Rohrabschnitt

- γ: Winkel zw. 133 und 134
- δ: Winkel zw. 153 und 154

## Patentansprüche

1. Atemstromsensor mit einem Strömungsrohr (12; 212), das eine Längsachse (14; 124; 214), einen ersten Strömungsrohrabschnitt (21; 81; 121; 221) und einen zweiten Strömungsrohrabschnitt (41; 101; 141; 241) aufweist, mit einem Strömungswiderstand (61), der in dem Strömungsrohr (12; 212) zwischen dem ersten Strömungsrohrabschnitt (21; 81; 121; 221) und dem zweiten Strömungsrohrabschnitt (41; 101; 141; 241) angeordnet ist, und mit Anschlüssen (32, 52; 82, 102; 132, 152; 232, 252) zur Abnahme der durch den Strömungswiderstand (61) erzeugten Druckdifferenz, wobei ein erster Anschluss (32; 82; 132; 232) über eine erste Verbindungsleitung in den ersten Strömungsrohrabschnitt (21; 81; 121; 221) mündet und ein weiterer Anschluss (52; 102; 152; 252) über eine zweite Verbindungsleitung in den zweiten Strömungsrohrabschnitt (41; 101; 141; 241) mündet, wobei die Anschlüsse (32, 52; 232, 252) jeweils einen Verbindungsleitungsabschnitt (33, 53; 83; 133, 153; 233; 253) der Verbindungsleitung aufweisen, der im Wesentlichen parallel zur Längsachse (14; 124; 214) des Strömungsrohrs (12; 212) verläuft, und die Öffnungen (35, 55; 85; 235, 255) der Anschlüsse (32, 52; 82, 102; 132, 152; 232, 252) in die gleiche Richtung ausgerichtet sind, **dadurch gekennzeichnet, dass** der erste Anschluss (32; 82; 132; 232) an dem ersten Strömungsrohrabschnitt (21; 81; 121; 221) und der weitere Anschluss (52; 102; 152; 252) an dem zweiten Strömungsrohrabschnitt (41; 101; 141; 241) angeordnet sind.

2. Atemstromsensor nach Anspruch 1, **dadurch gekennzeichnet, dass** die Verbindungsleitungen jeweils einen weiteren Verbindungsleitungsabschnitt (34, 54; 84; 134, 154; 234, 254) aufweisen, der zumindest bereichsweise gerade verläuft und einen Winkel (α, β; γ, δ) mit dem im Wesentlichen parallel zur Längsachse (14; 124; 214) des Strömungsrohrs (12; 212) verlaufenden Verbindungsleitungsabschnitt (33, 53; 83; 133, 153; 233, 253) einschliesst.

3. Atemstromsensor nach Anspruch 2, **dadurch gekennzeichnet, dass** der zumindest bereichsweise gerade verlaufende, weitere Verbindungsleitungsabschnitt (34; 154; 234; 254) der einen Verbindungsleitung mit dem im Wesentlichen parallel zur Längsachse (14; 124; 214) des Strömungsrohrs (12; 212) verlaufenden Verbindungsleitungsabschnitt (33; 153; 233; 253) dieser Verbindungsleitung einen Winkel (α) von 40° bis 70°, bevorzugt von 48° bis 62° und besonders bevorzugt von 52° bis 58°, einschliesst.

4. Atemstromsensor nach Anspruch 2 oder 3, **dadurch gekennzeichnet, dass** der zumindest bereichsweise gerade verlaufende, weitere Verbindungsleitungsabschnitt (54; 134; 234; 254) der anderen Verbindungsleitung mit dem im Wesentlichen parallel zur Längsachse (14; 124; 214) des Strömungsrohrs (12; 212) verlaufenden Verbindungsleitungsabschnitt (53; 133; 233; 253) der anderen Verbindungsleitung einen Winkel ((β) von 130° bis 160°, bevorzugt von 138° bis 152° und besonders bevorzugt von 142° bis 148°, einschliesst.

5. Atemstromsensor nach einem der Ansprüche 1 bis 4, **dadurch gekennzeichnet, dass** der erste Anschluss (32; 82; 132; 232) benachbart zum weiteren Anschluss (52; 102; 152; 252) angeordnet ist und bevorzugt der erste Anschluss (32; 82; 132; 232) auch beabstandet zum weiteren Anschluss (52; 102; 152; 252) angeordnet ist.

6. Atemstromsensor nach einem der Ansprüche 1 bis 5, **dadurch gekennzeichnet, dass** der erste Anschluss (32; 82; 132; 232) zumindest abschnittsweise an der äusseren Mantelfläche des ersten Strömungsrohrabschnitts (21; 81; 121; 221) angeordnet ist und/oder der weitere Anschluss (52; 102; 152; 252) zumindest abschnittsweise an der Mantelfläche des zweiten Strömungsrohrabschnitts (41; 101; 141; 241) angeordnet ist.

7. Atemstromsensor nach einem der Ansprüche 1 bis 6, **dadurch gekennzeichnet, dass** der erste Strömungsrohrabschnitt (21; 81; 121; 221) und/oder der zweite Strömungsrohrabschnitt (41; 101; 141; 241) einen im Wesentlichen zylindrischen Abschnitt (22, 42; 222, 242) und einen sich radial erweiternden Abschnitt (26; 226; 46, 246) aufweist, wobei sich der radial erweiternde Abschnitt (26; 226; 46, 246) zu einem offenen Ende (24, 224; 44, 244) des jeweiligen ersten Strömungsrohrabschnitts (21; 81; 121; 221) oder des zweiten Strömungsrohrabschnitts (41; 101; 141; 241) hin vergrössert, und insbesondere an dem offenen Ende (24, 224; 44, 244) des ersten Strömungsrohrabschnitts (21; 81; 121; 221) sowie des zweiten Strömungsrohrabschnitts (41; 101; 141; 241) jeweils ein radial abragender Flansch (27, 47; 227, 247) mit einer Anlagefläche vorgesehen ist.

8. Atemstromsensor nach Anspruch 7, **dadurch gekennzeichnet, dass** an dem sich radial erweiternden Abschnitt (26; 226) des ersten Strömungsrohrabschnittes (21; 81; 121; 221) eine erste Anschlussausnehmung (27a; 227a) zur zumindest abschnittsweisen Aufnahme des weiteren Anschlusses (52; 102; 152; 252) des zweiten Strömungsrohrabschnittes (41; 101; 141; 241) vorhanden ist und/oder an dem sich radial erweiternden Abschnitt (46; 246) des zweiten Strömungsrohrabschnittes eine weitere Anschlussausnehmung (47a; 247a) zur zumindest abschnittsweisen Aufnahme des ersten Anschlusses (32; 82; 132; 232) des ersten Strömungsrohrabschnittes (21; 81; 121; 221) vorhanden ist.

9. Atemstromsensor nach einem der Ansprüche 2 bis 5, **dadurch gekennzeichnet, dass** eine Werkzeugöffnung (36, 56; 236, 256) zur Entfernung eines Ausformwerkzeugs zur Ausformung des weiteren Verbindungsleitungsabschnitts (34, 54; 234, 254) einer Verbindungsleitung sowie jeweils ein Verschlusselement (38, 58; 238, 258) zum Verschliessen dieser Werkzeugöffnung (36, 56; 236, 256) vorgesehen ist, wobei das erste Verschlusselement (38; 238) zum Verschliessen des weiteren Verbindungsleitungsabschnitts (34; 234) der ersten Verbindungsleitung bevorzugt an dem ersten Strömungsrohrabschnitt (21; 221) angeordnet ist und wobei das zweite Verschlusselement (58; 258) zum Verschliessen des weiteren Verbindungsleitungsabschnitts (54; 254) der ersten Verbindungsleitung bevorzugt an dem zweiten Strömungsrohrabschnitt (41; 241) angeordnet ist.

10. Atemstromsensor nach Anspruch 9, **dadurch gekennzeichnet, dass** das erste Verschlusselement (38) über ein Scharnier, bevorzugt über ein Biegescharnier (39), an dem ersten Strömungsrohrabschnitt (21) verschwenkbar angeordnet ist und/oder das zweite Verschlusselement (58) über ein Scharnier, bevorzugt über ein Biegescharnier (59), an dem zweiten Strömungsrohrabschnitt (41) verschwenkbar angeordnet ist.

11. Atemstromsensor nach Anspruch 9 oder 10, **dadurch gekennzeichnet, dass** an den Werkzeugöffnungen (36, 56; 236, 256) und/oder den Verschlusselementen (38, 58; 238, 258) Nut-/Kammstrukturen (37, 40, 57, 60) vorgesehen sind und bevorzugt die Verschlusselemente (38, 58; 238, 258) mittels einer dichtend verbindenden Verbindung, besonders bevorzugt mittels einer Ultraschallverschweissung, zum Verschliessen der Werkzeugöffnungen (36, 56; 236, 256) an den jeweiligen Strömungsrohrabschnitt (21, 41; 221, 241) festgelegt sind.

12. Atemadapter mit zumindest mit einem Atemstromsensor (11) nach einem der Ansprüche 1 bis 11 und mit zumindest einem Verbindungsschlauch (73, 74), der einen der Anschlüsse (32, 52; 232, 252) des Atemstromsensors (11) mit einer Messeinrichtung (76) verbindet, zur Schaffung einer fluidschen Verbindung von der Messeinrichtung (76) zu dem Atemstromsensor (11), wobei die fluidische Verbindung direkt in einen der Strömungsrohrabschnitte (21; 81; 121; 221) des Atemstromsensors (11) stossstellenfrei ausgebildet ist.

13. Atemadapter nach Anspruch 12, **dadurch gekennzeichnet, dass** zumindest ein Beatmungsschlauch (77, 79) und ein Mundstück (78) vorgesehen ist, wobei der zumindest einen Beatmungsschlauch (77) das Mundstück (78) mit dem zumindest einen Atemstromsensor (11) verbindet.

14. Verfahren zur Herstellung eines Atemstromsensors (11) nach einem der Ansprüche 1 bis 11, **gekennzeichnet durch** die Schritte:
a) Ausformen des ersten Strömungsrohrabschnitts (21; 81; 121; 221) und des zweiten Strömungsrohrabschnitts (41; 101; 141; 241) jeweils in einer Herstellungswerkzeugform, wobei die Verbindungsleitungsabschnitte (33 bzw. 53; 83; 133 bzw. 153; 233 bzw. 253) und die weiteren Verbindungsleitungsabschnitte (34 bzw. 54; 84; 134 bzw. 154; 234 bzw. 254) der entsprechenden Verbindungsleitungen mittels Schiebern als Ausformwerkzeug und/oder mittels Pins als Ausformwerkzeug ausgebildet werden,
b) Entformen des ersten Strömungsrohrabschnitts (21; 81; 121; 221) und des zweiten Strömungsrohrabschnitts (41; 101; 141; 241) aus der jeweiligen Herstellungswerkzeugform,
c) Ausrichten des ersten Strömungsrohrabschnitts (21; 81; 121; 221) und des zweiten Strömungsrohrabschnitts (41; 101; 141; 241) und zwar axial sowie bezüglich der Winkellage zueinander, und
d) dichtendes Verbinden des ersten Strömungsrohrabschnitts (21; 81; 121; 221) und des zweiten Strömungsrohrabschnitts (41; 101; 141; 241).

## Claims

1. A respiratory flow sensor with a flow tube (12; 212), which has a longitudinal axis (14; 124; 214), a first flow tube portion (21; 81; 121; 221) and a second flow tube portion (41; 101; 141; 241), with a flow resistor (61), which is arranged in the flow tube (12; 212) between the first flow tube portion (21; 81; 121; 221) and the second flow tube portion (41; 101; 141; 241), and with ports (32, 52; 82, 102; 132, 152; 232, 252) for extracting the pressure difference generated by the flow resistor (61), wherein a first port (32; 82; 132; 232) opens into the first flow tube portion (21; 81; 121; 221) via a first connecting line and a further port (52; 102; 152; 252) opens into the second flow tube portion (41; 101; 141; 241) via a second connecting line, wherein the ports (32, 52; 232, 252) each have a connecting line portion (33; 53; 83; 133, 153; 233; 253) of the connecting line that runs essentially parallel to the longitudinal axis (14; 124; 214) of the flow tube (12; 212), and the openings (35, 55; 85; 235, 255) of the ports (32, 52; 82, 102; 132, 152; 232, 252) are aligned in the same direction, **characterized in that** the first port (32; 82; 132; 232) is arranged at the first flow tube portion (21; 81; 121; 221) and the further port (52; 102; 152; 252) is arranged at the second flow tube portion (41; 101; 141; 241).

2. The respiratory flow sensor according to Claim 1, **characterized in that** the connecting lines each comprise a further connecting line portion (34, 54; 84; 134, 154; 234, 254), which runs straight at least in sections and encloses an angle (α, β; γ, δ) with the connecting line portion (33, 53; 83; 133, 153; 233, 253) running essentially parallel to the longitudinal axis (14; 124; 214) of the flow tube (12; 212) .

3. The respiratory flow sensor according to Claim 2, **characterized in that** the further connecting line portion (34; 154; 234; 254) of the one connecting line running straight at least in sections encloses an angle (α) of 40° to 70°, preferably of 48° to 62° and particularly preferably of 52° to 58° with the connecting line portion (33; 153; 233; 253) of this connecting line running essentially parallel to the longitudinal axis (14; 124; 214) of the flow tube (12; 212) .

4. The respiratory flow sensor according to Claim 2 or 3, **characterized in that** the further connecting line portion (54; 134; 234; 254) of the other connecting line running straight at least in sections encloses an angle (β) of 130° to 160°, preferably of 138° to 152° and particularly preferably of 142° to 148° with the connecting line portion (53; 133; 233; 253) of the other connecting line running essentially parallel to the longitudinal axis (14; 124; 214) of the flow tube (12; 212).

5. The respiratory flow sensor according to one of Claims 1 to 4, **characterized in that** the first port (32; 82; 132; 232) is arranged adjacent to the further port (52; 102; 152; 252) and preferably the first port (32; 82; 132; 232) is also arranged spaced apart from the further port (52; 102; 152; 252).

6. The respiratory flow sensor according to one of Claims 1 to 5, **characterized in that** the first port (32; 82; 132; 232) is arranged at least in sections at the outer lateral surface of the first flow tube portion (21; 81; 121; 221) and/or the further port (52; 102; 152; 252) is arranged at least in sections at the lateral surface of the second flow tube portion (41; 101; 141; 241).

7. The respiratory flow sensor according to one of Claims 1 to 6, **characterized in that** the first flow tube portion (21; 81; 121; 221) and/or the second flow tube portion (41; 101; 141; 241) comprises an essentially cylindrical portion (22, 42; 222, 242) and a radially widening portion (26; 226; 46, 246), wherein the radially widening portion (26; 226; 46, 246) becomes larger towards an open end (24, 224; 44, 244) of the respective first flow tube portion (21; 81; 121; 221) or of the second flow tube portion (41; 101; 141; 241), and in particular a radially projecting flange (27, 47; 227, 247) with a contact face is provided in each case at the open end (24, 224; 44, 244) of the first flow tube portion (21; 81; 121; 221) and of the second flow tube portion (41; 101; 141; 241).

8. The respiratory flow sensor according to Claim 7, **characterized in that** a first port recess (27a; 227a) for receiving the further port (52; 102; 152; 252) of the second flow tube portion (41; 101; 141; 241) at least in sections is present at the radially widening portion (26; 226) of the first flow tube portion (21; 81; 121; 221) and/or a further port recess (47a; 247a) for receiving the first port (32; 82; 132; 232) of the first flow tube portion (21; 81; 121; 221) at least in sections is present at the radially widening portion (46; 246) of the second flow tube portion.

9. The respiratory flow sensor according to one of Claims 2 to 5, **characterized in that** a tool opening (36, 56; 236, 256) is provided for removing a moulding tool for the moulding of the further connecting line portion (34, 54; 234; 254) of a connecting line and in each case a closure element (38, 58; 238, 258) for closing this tool opening (36, 56; 236, 256), wherein the first closure element (38; 238) for closing the further connecting line portion (34; 234) of the first connecting line is preferably arranged at the first flow tube portion (21; 221) and wherein the second closure element (58; 258) for closing the further connecting line portion (54; 254) of the first connecting line is preferably arranged at the second flow tube portion (41; 241).

10. The respiratory flow sensor according to Claim 9, **characterized in that** the first closure element (38) is preferably arranged at the first flow tube portion (21) in a swivellable manner by means of a hinge, preferably by means of a living hinge (39), and/or the second closure element (58) is arranged at the second flow tube portion (41) in a swivellable manner by means of a hinge, preferably by means of a living hinge (59).

11. The respiratory flow sensor according to Claim 9 or 10, **characterized in that** groove/comb structures (37, 40, 57, 60) are provided at the tool openings (36, 56; 236, 256) and/or at the closure elements (38, 58; 238, 258) and preferably the closure elements (38, 58; 238, 258) are fixed at the respective flow tube portion (21, 41; 221, 241) for the closing of the tool openings (36, 56; 236, 256) by means of a connection that connects in a sealing manner, particularly preferably by means of an ultrasonic welding.

12. A respiration adapter with at least one respiratory flow sensor (11) according to one of Claims 1 to 11 and with at least one connecting tube (73, 74), which connects one of the ports (32, 52; 232, 252) of the respiratory flow sensor (11) to a measuring device (76), for the creation of a fluidic connection from the measuring device (76) to the respiratory flow sensor (11), wherein the fluidic connection is constituted free from butt joints directly into one of the flow tube portions (21; 81; 121; 221) of the respiratory flow sensor (11).

13. The respiration adapter according to Claim 12, **characterized in that** at least one breathing tube (77, 79) and a mouthpiece (78) is provided, wherein the at least one breathing tube (77) connects the mouthpiece (78) to the at least one respiratory flow sensor (11).

14. A method for the production of a respiratory flow sensor (11) according to one of Claims 1 to 11, **characterized by** the steps:
a) moulding of the first flow tube portion (21; 81; 121; 221) and the second flow tube portion (41; 101; 141; 241) each in a production tool mould, wherein the connecting line portions (33 or respectively 53; 83; 133 or respectively 153; 233 or respectively 253) and the further connecting line portions (34 or respectively 54; 84; 134 or respectively 154; 234 or respectively 254) of the corresponding connecting lines are formed as a moulding tool by means of sliders and/or as a moulding tool by means of pins,
b) removal of the first flow tube portion (21; 81; 121; 221) and the second flow tube portion (41; 101; 141; 241) from the respective production tool mould,
c) alignment of the first flow tube portion (21; 81; 121; 221) and the second flow tube portion (41; 101; 141; 241), and namely axially and with respect to the angular position to one another, and
d) sealing connection of the first flow tube portion (21; 81; 121; 221) and the second flow tube portion (41; 101; 141; 241).

## Revendications

1. Capteur de débit respiratoire comportant un tuyau d'écoulement (12 ; 212) qui présente un axe longitudinal (14 ; 124 ; 214), une première section de tuyau d'écoulement (21 ; 81 ; 121 ; 221) et une seconde section de tuyau d'écoulement (41 ; 101 ; 141 ; 241), une résistance d'écoulement (61) qui est disposée dans le tuyau d'écoulement (12 ; 212) entre la première section de tuyau d'écoulement (21 ; 81 ; 121 ; 221) et la seconde section de tuyau d'écoulement (41 ; 101 ; 141 ; 241), et des raccordements (32, 52 ; 82, 102 ; 132, 152 ; 232, 252) pour la réduction de la différence de pression générée par la résistance d'écoulement (61), un premier raccordement (32 ; 82 ; 132 ; 232) débouchant par une première conduite de liaison dans la première section de tuyau d'écoulement (21 ; 81 ; 121 ; 221) et un autre raccordement (52 ; 102 ; 152 ; 252) par une seconde conduite de liaison dans la seconde section de tuyau d'écoulement (41 ; 101 ; 141 ; 241), les raccordements (32, 52 ; 232, 252) présentant respectivement une section de conduite de liaison (33, 53 ; 83 ; 133, 153 ; 233 ; 253) de la conduite de liaison, laquelle section s'étend sensiblement parallèlement à l'axe longitudinal (14 ; 124 ; 214) du tuyau d'écoulement (12 ; 212), et les ouvertures (35, 55 ; 85 ; 235, 255) des raccordements (32, 52 ; 82 ; 102 ; 132 152 ; 232, 252) étant dirigées dans le même sens, **caractérisé en ce que** le premier raccordement (32, 82 ; 132, 232) est disposé au niveau de la première section de tuyau d'écoulement (21 ; 81 ; 121 ; 221) et l'autre raccordement (52 ; 102 ; 152 ; 252) au niveau de la seconde section de tuyau d'écoulement (41 ; 101 ; 141 ; 241).

2. Capteur de débit respiratoire selon la revendication 1, **caractérisé en ce que** les conduites de liaison présentent respectivement une autre section de conduite de liaison (34, 54 ; 84 ; 134, 154 ; 234, 254) qui s'étend tout droit du moins par endroits et circonscrit un angle (α, β, γ, δ) avec la section de conduite de liaison (33, 53 ; 83 ; 133, 153 ; 233 ; 253) s'étendant sensiblement parallèlement à l'axe longitudinal (14 ; 124 ; 214) du tuyau d'écoulement (12 ; 212).

3. Capteur de débit respiratoire selon la revendication 2, **caractérisé en ce que** l'autre section de conduite de liaison (34 ; 154 ; 234, 254) s'étendant tout droit du moins par endroits d'une conduite de liaison circonscrit avec la section de conduite de liaison (33 ; 153 ; 233 ; 253) s'étendant sensiblement parallèlement à l'axe longitudinal (14 ; 124 ; 214) du tuyau d'écoulement (12 ; 212) de cette conduite de liaison un angle (α) de 40° à 70°, de préférence 48° à 62° et en particulier de préférence 52° à 58°.

4. Capteur de débit respiratoire selon la revendication 2 ou 3, **caractérisé en ce que** l'autre section de conduite de liaison (34,; 134 ; 234 ; 254) s'étendant tout droit du moins par endroits de l'autre conduite de liaison circonscrit avec la section de conduite de liaison (53 ; 133 ; 233 ; 253) s'étendant sensiblement parallèlement à l'axe longitudinal (14 ; 124 ; 214) du tuyau d'écoulement (12 ; 212) de l'autre conduite de liaison un angle (β) de 130° à 160°, de préférence 38° à 152° et en particulier de préférence 142° à 148°.

5. Capteur de débit respiratoire selon une des revendications 1 à 4, **caractérisé en ce que** le premier raccordement (32 ; 82 ; 132 ; 232) est disposé à proximité de l'autre raccordement (52 ; 102 ; 152 ; 252) et que le premier raccordement (32 ; 82 ; 132 ; 232) est aussi disposé à distance de l'autre raccordement (52 ; 102 ; 152 ; 252).

6. Capteur de débit respiratoire selon une des revendications 1 à 5, **caractérisé en ce que** le premier raccordement (32 ; 82 ; 132 ; 232) est disposé du moins par endroits au niveau de la surface d'enveloppe extérieure de la première section de tuyau d'écoulement (21 ; 81 ; 121 ; 221) et/ou l'autre raccordement (52 ; 102 ; 152 ; 252) au moins par endroits au niveau de la surface d'enveloppe de la seconde section de tuyau d'écoulement (41 ; 101 ; 141 ; 241).

7. Capteur de débit respiratoire selon une des revendications 1 à 6, **caractérisé en ce que** la première section de tuyau d'écoulement (21 ; 81 ; 121 ; 221) et/ou la seconde section de tuyau d'écoulement (41 ; 101 ; 141 ; 241) présentent une section sensiblement cylindrique (22, 42 ; 222, 242) et une section s'élargissant radialement (26 ; 226 ; 46, 246), la section s'élargissant radialement (26 ; 226 ; 46, 246) s'agrandissant vers une extrémité ouverte (24, 224 ; 44, 244) de la première section de tuyau d'écoulement (21 ; 81 ; 121 ; 221) ou de la seconde section de tuyau d'écoulement (41 ; 101 ; 141 ; 241) respective, et une bride dépassant radialement (27, 47 ; 227, 247) dotée d'une surface d'appui étant prévue en particulier à l'extrémité ouverte (24, 224 ; 44, 244) de la première section de tuyau d'écoulement (21 ; 81 ; 121 ; 221) et de la seconde section de tuyau d'écoulement (41 ; 101 ; 141 ; 241).

8. Capteur de débit respiratoire selon la revendication 7, **caractérisé en ce que**, au niveau de la section s'élargissant radialement (26 ; 126) de la première section de tuyau d'écoulement (21 ; 81 ; 121 ; 221), il est prévu une première échancrure de raccordement (27a ; 227a) destinée à recevoir du moins par endroits l'autre raccordement (52 ; 102 ; 152 ; 252) de la seconde section de tuyau d'écoulement (41 ; 101 ; 141 ; 241) et/ou que, au niveau de la section s'élargissant radialement (46 ; 246) de la seconde section de tuyau d'écoulement, il est prévu une autre échancrure de raccordement (47a ; 247a) destinée à recevoir du moins par endroits le premier raccordement (32 ; 82 ; 132 ; 232) de la première section de tuyau d'écoulement (21 ; 81 ; 121 ; 221).

9. Capteur de débit respiratoire selon une des revendications 2 à 5, **caractérisé en ce qu'**il est prévu une ouverture d'outil (36, 56 ; 236, 256) pour enlever un outil de façonnage destiné à façonner l'autre section de conduite de liaison (34 ; 134 ; 234 ; 254) d'une conduite de liaison et respectivement un élément de fermeture (38, 58 ; 238, 258) pour fermer cette ouverture d'outil (36, 56 ; 236, 256), le premier élément de fermeture (38 ; 238) destiné à fermer l'autre section de conduite de liaison (34 ; 234) de la première conduite de liaison étant disposé de préférence au niveau de la première section de tuyau d'écoulement (21 ; 221) et le second élément de fermeture (58 ; 258) destiné à fermer l'autre section de conduite de liaison (54 ; 254) de la première conduite de liaison étant de préférence disposé au niveau de la seconde section de tuyau d'écoulement (41 ; 241).

10. Capteur de débit respiratoire selon la revendication 9, **caractérisé en ce que** le premier élément de fermeture (38) est disposé de manière à être pivotable au moyen d'une charnière, de préférence une charnière flexible (39), au niveau de la première section de tuyau d'écoulement (21) et/ou que le second élément de fermeture (58) est disposé de manière à être pivotable au moyen d'une charnière, de préférence une charnière flexible (59), au niveau de la seconde section de tuyau d'écoulement (41).

11. Capteur de débit respiratoire selon la revendication 9 ou 10, **caractérisé en ce que**, au niveau des ouvertures d'outil (36, 56 ; 236, 256) et/ou des éléments de fermeture (38, 58 ; 238, 258), des structures en rainure/peigne (37, 40, 57, 60) sont prévues et que les éléments de fermeture (38, 58 ; 238, 258) sont de préférence fixés au moyen d'une liaison à connexion étanche, de préférence au moyen d'un soudage aux ultrasons, pour fermer les ouvertures d'outil (36, 56 ; 236, 256) à la section de tuyau d'écoulement respective (21, 41 ; 221, 241).

12. Adaptateur respiratoire comportant au moins un capteur de débit respiratoire selon une des revendications 1 à 11 et au moins un tuyau de liaison (73, 74) qui connecte un des raccordements (32, 52 ; 232, 252) du capteur de débit respiratoire (11) à un dispositif de mesure (76) pour créer une liaison fluidique entre le dispositif de mesure (76) et le capteur de débit respiratoire (11), la liaison fluidique étant établie directement dans une des sections de tuyau d'écoulement (21 ; 81 ; 121 ; 221) du capteur de débit respiratoire (11) sans point de choc.

13. Adaptateur respiratoire selon la revendication 12, **caractérisé en ce qu'**il est prévu au moins un tuyau de ventilation (77, 79) et un embout (78), l'au moins un tuyau de ventilation (77, 79) connectant l'embout (78) à l'au moins un capteur de débit respiratoire (11).

14. Procédé de fabrication d'un capteur de débit respiratoire (11) selon une des revendications 1 à 11, **caractérisé par** les étapes suivantes :
a) façonnage de la première section de tuyau d'écoulement (21 ; 81 ; 121 ; 221) et de la seconde section de tuyau d'écoulement (41 ; 101 ; 141 ; 241) respectivement dans un moule d'outil de fabrication, les sections de conduite de liaison (33 ou 53 ; 83 ; 133 ou 153 ; 233 ou 253) et les autres sections de conduites de liaison (34 ou 54 ; 84 ; 134 ou 154 ; 234 ou 254) des conduites de liaison correspondantes étant réalisées au moyen de curseurs servant d'outil de façonnage et/ou au moyen de broches servant d'outil de façonnage,
b) formage de la première section de tuyau d'écoulement (21 ; 81 ; 121 ; 221) et de la seconde section de tuyau d'écoulement (41 ; 101 ; 141 ; 241) dans le moule d'outil de fabrication respectif,
c) orientation de la première section de tuyau d'écoulement (21 ; 81 ; 121 ; 221) et de la seconde section de tuyau d'écoulement (41 ; 101 ; 141 ; 241) et ce axialement et par rapport à la position angulaire mutuelle, et
d) connexion étanche de la première section de tuyau d'écoulement (21 ; 81 ; 121 ; 221) et de la seconde section de tuyau d'écoulement (41 ; 101 ; 141 ; 241) .
